(19) Europäisches Patentamt / European Patent Office / Office européen des brevets

(11) **EP 3 573 693 B1**

(12) **EUROPÄISCHE PATENTSCHRIFT**

(45) Veröffentlichungstag und Bekanntmachung des Hinweises auf die Patenterteilung:
**18.05.2022 Patentblatt 2022/20**

(21) Anmeldenummer: **18705094.3**

(22) Anmeldetag: **25.01.2018**

(51) Internationale Patentklassifikation (IPC):
**A61M 16/00** (2006.01)

(52) Gemeinsame Patentklassifikation (CPC):
**A61M 16/00; A61M 16/0051; A61M 16/024;**
A61M 2016/0027; A61M 2016/003;
A61M 2205/3334; A61M 2205/3355;
A61M 2205/502; A61M 2230/46

(86) Internationale Anmeldenummer:
**PCT/EP2018/051808**

(87) Internationale Veröffentlichungsnummer:
**WO 2018/138191 (02.08.2018 Gazette 2018/31)**

(54) **VORRICHTUNGEN UND VERFAHREN ZUR BEATMUNG EINES PATIENTEN**

DEVICES AND METHODS FOR VENTILATING A PATIENT

DISPOSITIFS ET PROCÉDÉS POUR LA VENTILATION ARTIFICIELLE D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.01.2017 DE 102017101645**

(43) Veröffentlichungstag der Anmeldung:
**04.12.2019 Patentblatt 2019/49**

(60) Teilanmeldung:
**22157329.8**

(73) Patentinhaber: **Ventinova Technologies B.V.**
**5652 BJ Eindhoven (NL)**

(72) Erfinder:
• **ENK, Dietmar**
**48653 Coesfeld (DE)**

• **BARNES, Thomas Heinrich**
**Warlingham**
**Surrey CR6 9LB (GB)**
• **VAN ASSELDONK, Dirk Theodorus Andreas**
**5464 PV Veghel (NL)**

(74) Vertreter: **karo IP**
**karo IP Patentanwälte**
**Kahlhöfer Rößler Kreuels PartG mbB**
**Postfach 32 01 02**
**40416 Düsseldorf (DE)**

(56) Entgegenhaltungen:
| | |
|---|---|
| EP-A1- 1 295 620 | EP-A2- 0 215 433 |
| US-A1- 2006 000 475 | US-A1- 2006 086 357 |
| US-A1- 2007 157 930 | US-A1- 2007 272 242 |
| US-A1- 2008 294 060 | US-A1- 2014 048 072 |
| US-B1- 6 390 091 | |

**Beschreibung**

Der Gegenstand der vorliegenden Erfindung bezieht sich auf eine Beatmungsvorrichtung zur Beatmung eines Patienten. Die Beatmungsvorrichtung umfasst zumindest eine Fluidzuführeinheit und zusätzlich eine Fluidabführeinheit, die zum Zuführen eines Fluids (insbesondere zumindest hauptsächlich Atemgas) in zumindest einen Atemweg, also in einen Lungenteil oder in die Lunge, eines Patienten und zum Abführen des Fluids aus diesem Atemweg geeignet ist.

Bei der Beatmung eines Patienten wird normalerweise eine Maske oder ein Tubus eingesetzt, über die bzw. den dem nach außen abgedichteten Atemweg mit niedrigem Druck ein Gas oder Gasgemisch, insbesondere Sauerstoff und Luft, zugeführt wird. Alternativ kann man ein solches Gas oder Gasgemisch aber auch mit hohem Druck und hohem Fluss stoßweise durch ein dünnes Lumen (ein Katheter oder eine Kanüle bzw. ein Tubus) in den nach außen offenen Atemweg injizieren (sogenannte Jet-Ventilation). Dieses Verfahren wird heute insbesondere bei diagnostischen und therapeutischen Eingriffen im Bereich des oberen Atemweges eingesetzt (endotracheale oder transtracheale Jet-Ventilation). Dabei kann dieses Verfahren in Notfallsituationen auch außerhalb von Krankenhäusern oder in stationären Situationen in Krankenhäusern angewendet werden.

Bei der transtrachealen Jet-Ventilation kann ein Patient durch einen durch die Haut direkt in die Luftröhre eingebrachten Katheter bzw. eine so platzierte Kanüle mit Sauerstoff bzw. einem Fluid versorgt werden. Diese Verfahren (trans- /endotracheal) sind Bestandteil der heute gültigen Algorithmen zum Management eines schwierigen Atemweges und insbesondere der Situation, in der ein Patient konventionell nicht zu beatmen bzw. zu intubieren ist (sogenannte "cannot ventilate, cannot intubate"-Situation).

Weiterhin sind aus der WO 2008/113752 A1 und WO 2015/004229 A1 jeweils Gasstromumkehr-Vorrichtungen bekannt, mit denen eine Beatmung (Inspiration und Exspiration) auch ausschließlich über ein Lumen erfolgen kann.

Künstliche oder mechanische Beatmung erfolgt entweder kontrolliert oder in Form von unterstützter Spontanatmung. Im ersten Fall hat das Beatmungsgerät (Respirator) die vollständige Kontrolle über das Atemmuster, während im zweiten Fall der zumindest teilweise spontan atmende Patient wesentlichen Einfluss auf das Atemmuster hat. Allen Formen der Beatmung ist aber gemeinsam, dass das Beatmungsgerät nahezu ausschließlich Einfluss auf die Einatemphase (Inspiration) nimmt. Die Exspiration kann - aus der Perspektive des Respirators - passiv erfolgen, d. h. die in den elastischen Gewebeelementen von Lunge und Thorax gespeicherte Energie treibt die Exspiration an.

Es sind verschiedene Beatmungsverfahren bekannt. Üblicherweise wird eine volumenkontrollierte Beatmung durchgeführt, wobei sämtliche Beatmungsparameter vorgegeben werden. Ziel- und Steuerungsparameter ist das Tidalvolumen (Atemzugvolumen $V_T$). Die resultierenden Atemwegsdrücke sind abhängig von den eingestellten Volumina sowie den pulmonalen Gegebenheiten des Patienten. Einstellparameter sind also Volumenstrom, Beatmungsfrequenz, PIP (peak inspiratory pressure und EEP (end-expiratory pressure, u. a. PEEP - positive end-expiratory pressure, ZEEP - zero end-expiratory pressure and NEEP - negative end-expiratory pressure). Im Folgenden wird immer auf den PEEP verwiesen. Der inspiratorische Höchstdruck (PIP) bezeichnet den höchsten positiven Druck, der während der Beatmung künstlich in dem Atemweg erzeugt wird. Dieser kann auch als Alarmgrenze vorgesehen sein, so dass ein Übersteigen dieses Druckwerts zu jedem Zeitpunkt möglichst verhindert wird. Der positive endexspiratorische Druck (PEEP) bezeichnet einen bei der Beatmung künstlich in dem Atemweg erzeugten positiven Druck, der nach Abschluss der Ausatmung (Exspiration) anliegt.

Bei der druckkontrollierten Beatmung wird ein anfangs hoher Volumenstrom kontinuierlich reduziert während ein Druckanstieg in dem Atemweg oder außerhalb des Atemwegs z. B. in der Beatmungsvorrichtung erfasst wird. Zielparameter und Kontrollvariable ist also der Druck. Eine Einstellung des Volumenstroms ist hierbei also nicht möglich, dieser wird aber erfasst und Alarmgrenzen können definiert werden.

Im Gegensatz zur Spontanatmung eines Patienten wird bei der künstlichen Beatmung das Fluid gegen die Elastizität des Atemwegs zugeführt. Durch die Erhöhung des Drucks im Brustraum verringern PEEP und PIP den Rückfluss des venösen Blutes zum Herzen, wodurch das Herzzeitvolumen sinken kann. Umgekehrt entsteht ein Rückstau in oberer und unterer Hohlvene mit entsprechenden Druckerhöhungen in vorgeschalteten Organen. Abhängig von der Höhe von PEEP und PIP kann es dadurch zu Schädigungen und Funktionseinschränkungen von Gehirn, Leber, Nieren und anderer Organe kommen.

Aus der US 2007/157930 A1 ist ein System zur Beatmung eines Patienten bekannt.

Hiervon ausgehend liegt der vorliegenden Erfindung die Zielsetzung zugrunde, eine verbesserte Beatmungsvorrichtung sowie ein verbessertes Beatmungsverfahren vorzuschlagen. Insbesondere soll die Beatmung möglichst individuell abgestimmt erfolgen, d. h. die Eigenschaften des zu beatmenden Patienten sollen vollumfänglich berücksichtigt werden. Weiterhin soll die Beatmung möglichst schonend erfolgen, eine Schädigung der Atemwege und anderer Organe soll in jedem Fall verhindert werden. Insbesondere soll eine Beatmungsvorrichtung vorgeschlagen werden, die es ermöglicht, eine derartige Beatmung durchzuführen.

Diese Zielsetzung wird durch eine Beatmungsvorrichtung mit den Merkmalen des Patentanspruchs 1 erreicht. Vorteilhafte Weiterbildungen und Ausgestaltungen der Beatmungsvorrichtungen und der Verfahren sind Gegenstand der jeweils abhängigen Ansprüche. Es ist darauf hinzuweisen, dass die in den abhängig formulierten Ansprüchen einzeln aufgeführten Merkmale in technologisch sinnvoller Weise miteinander kombiniert werden können und weitere Ausgestaltungen der Erfindung definieren. Darüber hinaus werden die in den Ansprüchen angegebenen Merkmale in der Beschreibung näher präzisiert und erläutert, wobei weitere bevorzugte Ausgestaltungen der Erfindung dargestellt werden.

Es wird eine (erste) Beatmungsvorrichtung zur Beatmung eines Patienten vorgeschlagen, zumindest umfassend eine Fluidzuführeinheit und eine Fluidabführeinheit, die zum Zuführen eines Fluids in zumindest einen Atemweg, also in einen Lungenteil oder in die Lunge, eines Patienten sowie zum Abführen des Fluids aus diesem Atemweg geeignet ist; sowie eine Steuereinrichtung. Die Steuereinrichtung ist, zumindest während eines Beatmungsvorgangs des zumindest einen Atemwegs, also dem zumindest einmaligen Zuführen des Fluids in den zumindest einen Atemweg und dem zumindest einmaligen Abführen des Fluids aus dem zumindest einen Atemweg durch Betreiben der Beatmungsvorrichtung, zur Einstellung eines Verlauf eines Druckes P [$cmH_2O$ oder millibar] in dem Atemweg und eines Verlauf eines dem Atemweg zugeführten und eines aus dem Atemweg abgeführten Volumens V [milliliter] des Fluids gemäß $V=f_{ZP}(P)$ und $V=f_{AP}(P)$ oder gemäß $P=f_{ZV}(V)$ und $P=f_{AV}(V)$ eingerichtet, wobei der Beatmungsvorgang innerhalb eines Druckintervalls stattfindet; wobei durch die Steuereinrichtung der Beatmungsvorgang so einstellbar ist, dass

a) über zumindest 60 %, insbesondere zumindest 80 %, des Druckintervalls ein Verhältnis aus

- einem Betrag eines Maßes der Veränderung des bei einem Druck $P_0$ vorliegenden ersten Volumens während des Zuführens des Fluids, also $df_{AP}/d(P) (P_0)$ und
- einem Betrag eines Maßes der Veränderung eines bei demselben Druck $P_0$ vorliegenden zweiten Volumens während des Abführens des Fluids, also $df_{ZP}/d(P) (P_0)$

oder

b) über zumindest 60 %, insbesondere zumindest 80 %, des Volumenintervalls ein Verhältnis aus

- einem Betrag eines Maßes der Veränderung des bei einem Volumen $V_0$ vorliegenden ersten Drucks während des Zuführens des Fluids, also $df_{AV}/d(V) (V_0)$ und
- einem Betrag eines Maßes der Veränderung eines bei demselben Volumen $V_0$ vorliegenden zweiten Drucks während des Abführens des Fluids, also $df_{ZV}/d(V) (V_0)$

einen Wert von mindestens 0,5 und höchstens 2,0 aufweist.

Insbesondere wird das Volumen [Milliliter] über die Steuereinrichtung ermittelt. Dabei wird eine Zuführrate bzw. eine Abführrate des Fluids (also eine Volumenstromrate in Milliliter pro Zeiteinheit) gemessen bzw. überwacht. Insbesondere wird der in dem Atemweg vorliegende Druck [$cmH_2O$ oder Millibar] über einen Drucksensor überwacht, wobei die Steuereinrichtung diesen Wert für den Druck verarbeitet.

Das Maß der Veränderung des bei einem Druck $P_0$ vorliegenden ersten Volumens ist z. B. die Steigung einer Volumen-Druck Kurve in einem Volumen-Druck Diagramm. Dabei wird entlang der einen Achse der Druck und entlang der anderen Achse das Volumen aufgetragen. Dabei weist die Kurve einen ersten Kurvenabschnitt $V=f_{ZP}(P)$ bzw. $P=f_{ZV}(V)$ und einen zweiten Kurvenabschnitt $V=f_{AP}(P)$ bzw. $P=f_{AV}(V)$ auf, wobei der erste Kurvenabschnitt den Verlauf des zugeführten Volumens V und des Druckes P während des Zuführens des Fluids in den zumindest einen Atemweg und der zweite Kurvenabschnitt den Verlauf des abgeführten Volumens V und des Druckes P während des Abführens des Fluids aus dem zumindest einen Atemweg darstellt. Die Steigung wird durch die erste Ableitung der jeweiligen Funktion $V=f_{ZP}(P)$ (bzw. $P=f_{ZV}(V)$) und $V=f_{AP}(P)$ (bzw. $P=f_{AV}(V)$) ermittelt, also $df_{AP}/d(P) (P_0)$ und $df_{ZP}/d(P) (P_0)$. Hier wird also durch die Steuereinrichtung der Beatmungsvorgang so eingestellt, dass über zumindest 60 %, insbesondere zumindest 80 %, des Druckintervalls ein Verhältnis aus einem Betrag einer ersten Steigung des ersten Kurvenabschnitts bei einem Druck $P_0$, also $df_{ZP}/d(P) (P_0)$ und einem Betrag einer zweiten Steigung des zweiten Kurvenabschnitts, also $df_{AP}/d(P) (P_0)$, bei demselben Druck $P_0$, einen Wert von mindestens 0,5 und höchstens 2,0 aufweist. Der Betrag des Ergebnisses der Gleichung $[df_{AP}/d(P) (P_0)] / [df_{ZP}/d(P) (P_0)]$ soll also mindestens 0,5 und höchstens 2,0 betragen. Insbesondere ist $P_0$ also jeder Druck innerhalb des Druckintervalls bzw. innerhalb eines 60 %-igen bzw. 80 %-igen Anteils des Druckintervalls. Die obigen Ausführungen gelten gleichermaßen für die Verläufe der Funktionen $P=f_{ZV}(V)$ und $P=f_{AV}(V)$.

Der Betrag gibt den Wert des Ergebnisses bzw. des Maßes der Veränderung und der Steigung unabhängig von dessen Vorzeichen an.

Insbesondere wird vorgeschlagen, dass die Steuereinrichtung zur Ermittlung eines Verlaufs des Druckes P in dem Atemweg und eines Verlaufs eines dem Atemweg zugeführten und eines aus dem Atemweg abgeführten Volumens V des Fluids für eine Compliance des Patienten gemäß einem von $V=f_{CP}(P)$ und $P=f_{CV}(V)$ eingerichtet ist; wobei der Beatmungsvorgang so einstellbar ist, dass

a) über zumindest 60 %, insbesondere zumindest 80 %, des Druckintervalls ein Verhältnis aus

- jedem von $df_{AP}/d(P) (P_0)$, $df_{ZP}/d(P) (P_0)$ und
- einem Betrag eines Maßes der Veränderung des bei einem Druck $P_0$ vorliegenden ersten Vo-

lumens der Compliance, also $df_{CP}/d(P)$ $(P_0)$, oder

b) über zumindest 60 %, insbesondere zumindest 80 %, des Volumenintervalls ein Verhältnis aus

- jedem von $df_{AV}/d(V)$ $(V_0)$, $df_{ZV}/d(V)$ $(V_0)$ und
- und einem Betrag eines Maßes der Veränderung des bei einem Volumen $V_0$ vorliegenden ersten Drucks der Compliance, also $df_{CV}/d(V)$ $(V_0)$

einen Wert von mindestens 0,5 und höchstens 2,0 aufweist.

Insbesondere ist der Beatmungsvorgang so einstellbar, dass über zumindest 60 %, insbesondere zumindest 80 %, des Druckintervalls oder des Volumenintervalls das Verhältnis einen Wert von mindestens 0,67 und höchstens 1,5 aufweist.

Bevorzugt ist der Beatmungsvorgang so einstellbar, dass über zumindest 60 %, insbesondere zumindest 80 %, des Druckintervalls oder des Volumenintervalls das Verhältnis größer oder kleiner als 1,0 ist, insbesondere größer als 1,1 oder kleiner als 0,9.

Der Beatmungsvorgang wird insbesondere am Anfang der Zuführung des Fluids und am Anfang der Abführung sowie am Ende der Zuführung und am Ende der Abführung so eingestellt, dass hier die genannten Verhältnisse vorliegen (also z. B. ausgehend von diesen Punkten über jeweils 30 %, bzw. jeweils 40 % des Druckintervalls oder des Volumenintervalls, so dass diese Verhältnisse über insgesamt 60 % bwz. 80 % des Druckintervalls oder des Volumenintervalls vorliegen).

Der Grundgedanke der Erfindung ist es, die Beatmung des Patienten mit einem möglichst geringen Energieeintrag, bzw. einer geringen Absorption von Energie durch den Atemweg (im Folgenden auch als Verlustenergie E bezeichnet) durchzuführen. Ein geringer Energieeintrag in die Atemwege des Patienten bedeutet auch eine geringstmögliche Schädigung der Atemwege und weiterer Organe des Patienten.

Eine solche Minimierung des Energieeintrags (der Verlustenergie E) wird insbesondere dadurch erreicht, dass der Beatmungsvorgang vollständig hinsichtlich der Zuführung und Abführung von Fluid in und aus dem zumindest einen Atemweg gesteuert und kontrolliert wird. Insbesondere wird also zu insbesondere jedem Zeitpunkt die Fluidzuführrate und Fluidabführrate durch die Steuereinrichtung bestimmt, kontrolliert und geregelt.

Diese Steuerung und Kontrolle des Beatmungsvorgangs (also Zuführung und Abführung von Fluid in und aus dem Atemweg) erfolgt insbesondere entlang einer in einem Volumen-Druck Diagramm darstellbaren Compliance-Kurve des Atemwegs eines Patienten. Diese Compliance-Kurve verdeutlicht ein (minimales) Druckintervall, ggf. ausgehend von einem vorgegebenen PEEP oder PIP, in dem ein vorgegebenes Volumen des Fluids zu- und abgeführt wird. Die Beatmung des Patienten soll nun während der Zuführung und während der Abführung des Fluids so erfolgen, dass eine Volumen-Druck Kurve des jeweiligen Beatmungsvorgangs möglichst nah an der Compliance-Kurve verläuft. Allerdings wurde nun herausgefunden, dass zur Minimierung des Energieeintrags auch weitere Faktoren zu berücksichtigen sind. Diese können jeweils einzeln aber selbstverständlich auch (gerade) in Kombination mit den jeweils anderen Faktoren kombiniert werden.

Bei den hier vorgeschlagenen Vorrichtungen und Verfahren gilt insbesondere, dass dem Bediener der Vorrichtungen bzw. durch das Verfahren zumindest einer, ggf. mehrere oder sogar alle, der folgenden Parameter (z. B. aufgrund von Erfahrungswerten, Patientendaten, der Compliance des Atemwegs, des Widerstands des zumindest einen Atemwegs (z. B. durch Plethysmographie ermittelbar) etc.) bzw. Werte für die Parameter vorgeschlagen oder vorgegeben werden, bei denen die Beatmung des Patienten erfolgen soll: PEEP [Zentimeter Wassersäule - $cmH_2O$, oder Millibar], PIP [Zentimeter Wassersäule - $cmH_2O$, oder Millibar], Volumenflussrate [Milliliter/Minute], Verhältnis zwischen Dauer der Zuführung des Fluids und Dauer der Abführung des Fluids aus dem Atemweg - also I/E (duration inspiration/ duration expiration).

Insbesondere wird ein Verhältnis I/E von 1:1 vorgeschlagen, wobei Abweichungen von bis zu 20 % insbesondere möglich sind, wobei die Exspiration auch länger andauern kann, insbesondere bis zu einem Verhältnis von 1:1,5. Eine weitere Abweichung wird insbesondere nur dann vorgenommen, wenn dieses Verhältnis am Patienten nicht anwendbar ist (z. B. aufgrund von Krankheiten, Abnormitäten, etc.).

Es wurde nun insbesondere festgestellt, dass die erste Steigung eines ersten Kurvenabschnitts einer Volumen-Druck Kurve, darstellbar in einem Volumen-Druck Diagramm, und die zweite Steigung eines zweiten Kurvenabschnitts, bei einem jeweils gleichen Druck $P_0$ (der Druck $P_0$ liegt innerhalb des Druckintervalls) in einem möglichst großen Bereich des Druckintervalls möglichst gleich groß sein sollte. Gleiches gilt für ein Volumen $V_0$, wobei die erste Steigung eines ersten Kurvenabschnitts einer Volumen-Druck Kurve, darstellbar in einem Volumen-Druck Diagramm, und die zweite Steigung eines zweiten Kurvenabschnitts, bei einem jeweils gleichen Volumen $V_0$ (das Volumen $V_0$ liegt innerhalb des Volumenintervalls) in einem möglichst großen Bereich des Volumenintervalls möglichst gleich groß sein sollte.

Dabei wird durch die Steuereinrichtung zum einen der Druck- und der Volumenverlauf während der Zuführung von Fluid in den zumindest einen Atemweg gesteuert und kontrolliert. In Abhängigkeit von dem Verlauf dieses ersten Kurvenabschnitts wird nun aber auch die Abführung des Fluids aus dem Atemweg gesteuert und kontrolliert. Hier wird nun insbesondere keine passive Exspiration zugelassen, die üblicherweise einen von dem ersten Kurvenabschnitt stark abweichenden zweiten Kurvenabschnitt erzeugen würde. Im Gegenteil, es wird vorgeschlagen auch die Abführung des Fluids aktiv durch die Steuereinrichtung zu kontrollieren und zu steuern, wobei der zweite Kurvenabschnitt dem Verlauf des ersten Kurvenabschnitts angenähert wird.

Erste Versuche mit derartigen Beatmungsvorrichtungen und Verfahren haben ergeben, dass durch eine derartige Steuerung der Beatmung eine Schädigung des zumindest einen Atemwegs (VILI- ventilator induced lung injury) zumindest verringert oder sogar effektiv verhindert werden kann.

Insbesondere wird vorgeschlagen, während einer Beatmung des zumindest einen Atemwegs des Patienten die Compliance C des Atemwegs zu bestimmen und die Beatmung unter Berücksichtigung der ermittelten Compliance durchzuführen. Die Ermittlung oder die zusätzliche Abschätzung eines Verlaufs zumindest eines Teilbereichs einer Compliance-Kurve erfolgt durch das Zuführen und/oder Abführen des Fluids aus dem zumindest einen Atemweg und durch Ermittlung zumindest eines Wertes der Compliance. Für die Compliance C gilt: C = delta V / delta P [Milliliter/Millibar oder Milliliter/$cmH_2O$].

Die Compliance gibt an, wie viel Fluid, also ein Volumen delta V [Milliliter], in den mindestens einen Atemweg eingeführt oder aus dem Atemweg entfernt wird, so dass sich ein Druck in dem Atemweg um eine Druckdifferenz delta P [Millibar] verändert. Die Steuereinrichtung bestimmt, unter Berücksichtigung des ermittelten oder zusätzlich abgeschätzten Verlaufs des zumindest einen Teilbereichs der Compliance-Kurve, eine Lage eines Druckintervalls mit den Drücken P1 und P2 und stellt diese Drücke an der Beatmungsvorrichtung ein (z. B. PEEP als P1 und PIP als P2), so dass zumindest ein Beatmungsvorgang, also eine Inspiration und/oder eine Exspiration, zwischen diesen Drücken P1 und P2 erfolgt und ein Betrag der Compliance dieses Beatmungsvorgangs möglichst groß ist.

Eine Minimierung des Energieeintrags wird durch die Ermittlung des geringstmöglichen Druckes erreicht, bei dem ein benötigtes Atemzugvolumen $V_T$ (Tidalvolumen) dem Patienten zugeführt werden kann. Diese Drücke P1 und P2 des Druckintervalls werden insbesondere anhand der jeweils vorliegenden Compliance des beatmeten Patienten ermittelt.

In diesem Zusammenhang wird darauf hingewiesen, dass allgemein zwei Arten der Compliance bekannt sind, einerseits die statische Compliance und andererseits die dynamische Compliance. Zur Ermittlung der statischen Compliance wird ein (festgelegtes) Volumen an Fluid, ausgehend von einem Druck P1, einem Atemweg zugeführt. Daraufhin wird dieser Zustand gehalten, bis sich ein Druck P2 einstellt. Demgegenüber wird die dynamische Compliance während einer fortlaufenden Beatmung ermittelt. Nach dem Zuführen des (festgelegten) Volumens an Fluid wird unmittelbar daran anschließend der dann vorliegende Druck P2 gemessen. Das Druckintervall P1, P2 ist daher bei der dynamischen Compliance regelmäßig größer oder gleich groß wie das Druckintervall bei der statischen Compliance. Dabei wird die Compliance als eine Kurve in einem Druck-Volumen Diagramm (oder Volumen-Druck Diagramm dargestellt, da die Compliance im Allgemeinen ein sich - mit sich änderndem Druck - änderndes Verhältnis von V/P aufweist.

Insbesondere wird also während mindestens eines Beatmungsvorganges (Inspiration - also Zuführen von Fluid - und Exspiration - also Abführen von Fluid aus dem Atemweg) der Verlauf der Compliance-Kurve ermittelt oder zusätzlich abgeschätzt (z. B. aufgrund von Erfahrungswerten). Dabei wird insbesondere gerade der Teilbereich der Compliance-Kurve ermittelt, bei dem ein bestimmtes Volumen V (ggf. $V_T$) in einem möglichst kleinen Druckintervall zugeführt werden kann.

Insbesondere wird zur Ermittlung des Verlaufs der Compliance-Kurve ein Volumen an Fluid, bevorzugt ein kleines Volumen delta V von höchstens 100 ml, besonders bevorzugt von höchstens 50 ml, über die Fluidzuführeinheit dem mindestens einen Atemweg zugeführt. Während und/oder bevorzugt nach Zuführung dieses Volumens wird die Druckänderung delta P in dem mindestens einen Atemweg gemessen und ein Wert für die Compliance ermittelt. Entweder wird nun bereits unter Berücksichtigung von Erfahrungswerten oder ggf. unter Berücksichtigung von bereits vorher ermittelten Werten für die Compliance dieses Patienten zumindest der Verlauf des Teilbereichs der Compliance-Kurve abgeschätzt. Oder es werden weitere (kleine) Volumina delta V zugeführt und die jeweilige Druckänderung delta P ermittelt. Aus diesen Werten für die Compliance kann (mit steigender Genauigkeit) der Verlauf zumindest des Teilbereichs der Compliance-Kurve bestimmt und/oder abgeschätzt werden. Weiterhin kann aufgrund von fallenden oder steigenden Beträgen der Werte der Compliance der Verlauf der Compliance-Kurve und die bevorzugte Lage eines, für die folgende Beatmung des Patienten vorgesehenen Druckintervalls mit den Drücken P1 (insbesondere PEEP) und P2 (insbesondere PIP) bestimmt bzw. abgeschätzt werden.

In einem Druck-Volumen Diagramm gilt $V=f_{CP}(P)$ und in einem Volumen-Druck Diagramm $P=f_{CV}(V)$, wobei $V=f_{CP}(P)$ und $P=f_{CV}(V)$ jeweils die Funktion darstellen, durch die die Kurve (also die Compliance) in dem jeweiligen Diagramm beschrieben wird.

Insbesondere kann zumindest eine der folgenden Größen: PEEP, Atemfrequenz, Volumenstromrate, I/E, Widerstand des zumindest einen Atemwegs voreingestellt oder vorher ermittelt werden, so dass das benötigte Atemzugvolumen $V_T$, unter der Randbedingung eines möglichst geringen Energieeintrags, zugeführt werden kann. Bevorzugt kann auch jede dieser Größen nach Ermittlung und Bewertung des Beatmungsvorganges weiter angepasst werden, so ein vorbestimmtes Atemzugvolumen $V_T$ unter den dann eingestellten Parametern zugeführt wird.

Eine Fluidzuführeinheit und eine Fluidabführeinheit umfasst zumindest eine (gemeinsame) Druckgasquelle bzw. eine Einrichtung, mit der ein Fluid (z. B. ein Gas bzw. ein Gasgemisch, geeignet zur Sicherstellung der Beatmung eines Patienten) in den bzw. aus dem mindestens einen Atemweg des Patienten eingeführt bzw. ausgeführt werden kann. Bevorzugt ist ausschließlich eine Druckgasquelle vorhanden, bzw. erfolgt auch die Ausatmung über eine Beatmungsvorrichtung, z. B. eine eingangs erwähnte Gasstromumkehr-Vorrichtung, wobei das Fluid über ein Lumen dem Atemweg zugeführt und über ggf. das gleiche Lumen wieder abgeführt wird.

Die Steuereinrichtung ist insbesondere zur Ermittlung oder zusätzlich zum Abschätzen eines Verlaufs zumindest eines Teilbereichs einer Compliance-Kurve geeignet. Die Ermittlung der Compliance-Kurve erfolgt während einer Beatmung durch das Zuführen und/oder Abführen des Fluids aus dem zumindest einen Atemweg und durch Ermittlung zumindest eines Wertes der Compliance. Der Verlauf der Compliance-Kurve eines Patienten ist insbesondere unter Berücksichtigung des mindestens einen Wertes der Compliance abschätzbar.

Die Compliance kann insbesondere in zeitlichen Abständen oder nach einer gewissen Anzahl von Beatmungen wiederholend ermittelnd werden.

Insbesondere greift die Steuereinrichtung dabei auf die Messwerte mindestens eines Drucksensors zurück und überwacht die über die Fluidzuführeinheit zugeführten bzw. über die Fluidabführeinheit abgeführten Volumenströme.

Insbesondere wird der in dem Atemweg jeweils vorliegende Druck überwacht und/oder gemessen bzw. rechnerisch abgeschätzt bzw. ermittelt. Bevorzugt ist also ein Drucksensor in dem Atemweg angeordnet, so dass insbesondere auch eine kontinuierliche Druckmessung in dem Atemweg auch während der Beatmung möglich ist. Der Drucksensor kann ebenfalls außerhalb des Atemwegs an der Fluidzuführeinheit oder der Fluidabführeinheit angeordnet sein.

Eine derartige Anordnung eines Drucksensors ist insbesondere bei der Ermittlung des Verlaufs der Compliance-Kurve vorteilhaft, da hier bei der kontinuierlichen, oder in Teilschritten erfolgenden Zufuhr eines Volumens oder von Teilvolumina des Fluids der sich (jeweils) ändernde Druck delta P in dem Atemweg bestimmt werden kann.

Bevorzugt erfolgt während der Beatmung des Patienten eine kontinuierliche (also zu jedem Zeitpunkt stattfindende) aktive Steuerung des während der Inspiration der Lunge des Patienten zugeführten und während der Exspiration aus der Lunge des Patienten abgeführten Fluids durch die Beatmungsvorrichtung. Die aktive Steuerung umfasst eine kontinuierliche Druckänderung des zugeführten und abgeführten Fluids durch die Beatmungsvorrichtung. Der kontinuierlich geänderte Druck ist insbesondere der Druck innerhalb des zumindest einen Atemwegs und damit insbesondere innerhalb der Lunge. Dieser Druck kann über eine Messung am Ende einer, in den Atemweg hinreichenden Beatmungsvorrichtung, z. B. einem Katheter, über einen Sensor bestimmt werden.

Die kontinuierliche Druckänderung führt insbesondere zu einer kontinuierlichen Steuerung der Fluidzufuhrrate und Fluidabfuhrrate [Milliliter/Sekunde] durch die Beatmungsvorrichtung hin zu der Lunge bzw. aus der Lunge während der Beatmungsvorgänge. Insbesondere wird so das in der Lunge vorhandene Fluidvolumen (Volumen V) kontinuierlich geändert. Bevorzugt werden während der Änderung des in der Lunge befindlichen Fluidvolumens die Fluidzufuhrrate und/oder die Fluidabfuhrrate durch die Beatmungsvorrichtung zu der Lunge hin bzw. aus der Lunge nicht verändert und bleibt entsprechend im Wesentlichen konstant. Dabei muss die Fluidzufuhrrate nicht zwingend der Fluidabfuhrrate entsprechen, sie kann aber auch betragsgleich sein. Weiter kann die Fluidzufuhrrate von einem Inspirationsvorgang zum folgenden Inspirationsvorgang variiert werden. Gleiches gilt - insbesondere davon unabhängig - für die Fluidabfuhrrate während aufeinanderfolgender Exspirationsvorgänge.

Insbesondere werden Zustände, in denen innerhalb eines Zeitintervalls keine Änderung des Drucks und insbesondere keine Änderung des in der Lunge vorhandenen Fluidvolumens erfolgen, vermieden. Bevorzugt sind solche Zeitintervalle, in denen keine Änderung des Drucks und/oder insbesondere keine Änderung des in der Lunge vorhandenen Fluidvolumens erfolgen, höchstens 0,5 s [Sekunden], insbesondere höchstens 0,2 s, bevorzugt höchstens 0,1 s lang und betreffen insbesondere (ausschließlich) den Zeitpunkt der Fluidstromumkehr (also Übergang von Fluidzufuhr zu Fluidabfuhr und umgekehrt).

Der Druck wird insbesondere in dem Patienten selbst gemessen, besonders vorteilhaft im Bereich der Ausströmung aus der Beatmungsvorrichtung, also aus einem das Fluid transportierenden Lumen (Tubus/Katheter), in den Atemweg des Patienten. Alternativ und/oder zusätzlich wird der Druck in der Beatmungsvorrichtung gemessen.

Der Druck in der Beatmungsvorrichtung entspricht insbesondere nicht dem Druck in dem Atemweg des Patienten. Insbesondere kann eine kontinuierliche Änderung des Drucks in den Atemwegen auch durch einen zumindest zeitweisen konstanten Druck in der Beatmungsvorrichtung eingestellt werden.

Eine Änderung des Drucks in den Atemwegen kann insbesondere auch dann noch gemessen werden, wenn eine Fluidzufuhrrate oder Fluidabfuhrrate Null beträgt. Diese Änderung resultiert insbesondere aus den Eigenschaften der Atemwege selbst. Eine Fluidzufuhrrate und Fluidabfuhrrate von Null soll möglichst vermieden werden (höchstens für Zeitintervalle von bis zu 0,5 s, insbesondere höchstens 0,2 s oder 0,1 s, und auch dann nur zum Zeitpunkt der Fluidstromumkehr; ggf. sind längere Zeitintervalle von bis zu 2,0 s möglich, um z. B. eine Druckmessung durchzuführen, wobei ein solches verlängertes Zeitintervall nur in Abständen von mindestens 30 s, insbesondere mindestens 2 Minuten, bevorzugt mindestens 5 Minuten vorgesehen wird). Die Fluidzufuhrrate und Fluidabfuhrrate wird dazu insbesondere (ausschließlich) durch die Beatmungsvorrichtung vorgegeben, wobei der Druck in den Atemwegen überwacht wird.

Insbesondere wird so ein sinusförmiges oder sägezahnartiges Beatmungsmuster (Druck [Millibar] über der Zeit [Sekunde]) eingestellt, wobei eine Steigung der Kurve (Druck über der Zeit) ständig ungleich Null ist und insbesondere nur zu dem Zeitpunkt der Fluidstromumkehr für ein Zeitintervall von höchstens 0,5 s [Sekunden], insbesondere höchstens 0,2 s, bevorzugt höchstens 0,1 s, besonders bevorzugt nie, eine Steigung von Null aufweist.

Insbesondere wird dem Patienten bevorzugt zu allen Zeitpunkten der Beatmung durch die Beatmungsvorrichtung ein Beatmungsmuster vorgegeben, d. h. die Fluidzufuhrrate (inspiratorischer Fluss) und Fluidabfuhrrate (exspiratorischer Fluss) wird (allein) durch die Beatmungsvorrichtung (und nicht durch den Patienten) gesteuert und bestimmt.

Insbesondere erfolgt die Fluidzufuhr und ggf. zusätzlich die Fluidabfuhr ausschließlich über die Beatmungsvorrichtung bzw. über mindestens ein, in die Atemwege des Patienten eingeführtes Lumen.

Die kontinuierliche Druckänderung stellt sicher, dass die Fluidzufuhr und Fluidabfuhr nicht zu schnell oder zu langsam erfolgt und kann so eine Schädigung der Atemwege und insbesondere des Lungengewebes verhindern oder zumindest minimieren.

Weiterhin kann die Fluidzufuhr und Fluidabfuhr, z. B. unter Berücksichtigung einer Compliance der Atemwege, in vorteilhaften Druckintervallen (also zwischen einem ersten höheren Druck und einem zweitem niedrigeren Druck) und in einer vorbestimmbaren Beatmungsfrequenz erfolgen.

Hinsichtlich der Bestimmung der Compliance und einer Beatmungsvorrichtung sowie eines Verfahrens zum Betreiben einer Beatmungsvorrichtung, die jeweils die Compliance bei der Beatmung berücksichtigen, wird auf die bisher unveröffentlichte DE 10 2016 109 528.1 verwiesen.

Insbesondere ist der Beatmungsvorgang so einstellbar, dass

a) während des Zuführens des Fluids ein bei dem Druck $P_0$ vorliegendes erstes Volumen und während des Abführens des Fluids ein bei demselben Druck $P_0$ vorliegendes zweites Volumen sich höchstens um 30 %, insbesondere um höchstens 20 %, bevorzugt um höchstens 10 %, des in dem Druckintervall vorliegenden Volumenintervalls unterscheidet oder

b) während des Zuführens des Fluids ein bei dem Volumen $V_0$ vorliegender erster Druck und während des Abführens des Fluids ein bei demselben Volumen $V_0$ vorliegender zweiter Druck sich höchstens um 30 %, insbesondere um höchstens 20 %, bevorzugt um höchstens 10 %, des in dem Volumenintervall vorliegenden Druckintervalls unterscheidet.

Bevorzugt ist

a) der Beatmungsvorgang über zumindest 60 %, insbesondere zumindest 80 %, des Druckintervalls so einstellbar, dass während des Zuführens des Fluids ein bei dem Druck $P_0$ vorliegendes erstes Volumen und während des Abführens des Fluids ein bei demselben Druck $P_0$ vorliegendes zweites Volumen sich mindestens um 1 %, bevorzugt mindestens um 3 %, des in dem Druckintervall vorliegenden Volumenintervalls unterscheidet oder

b) der Beatmungsvorgang über zumindest 60 %, insbesondere zumindest 80 % des Volumenintervalls so einstellbar, dass während des Zuführens des Fluids ein bei dem Volumen $V_0$ vorliegender erster Druck und während des Abführens des Fluids ein bei demselben Volumen $V_0$ vorliegender zweiter Druck sich mindestens um 1 %, bevorzugt mindestens um 3 %, des in dem Volumenintervall vorliegenden Druckintervalls unterscheidet.

Insbesondere ist die Steuereinrichtung

a) zur Bestimmung von Integralen von $f_{ZP}(P)$ und $f_{AP}(P)$ in dem Druckintervall sowie zur Bestimmung einer Differenz von $\int f_{ZP}(P)\,dP$ und $\int f_{AP}(P)\,dP$ in dem Druckintervall oder

b) zur Bestimmung von Integralen von $f_{ZV}(V)$ und $f_{AV}(V)$ in dem Volumenintervall sowie zur Bestimmung einer Differenz von $\int f_{ZV}(V)\,dV$ und $\int f_{AV}(V)\,dV$ in dem Volumenintervall

geeignet.

Insbesondere wird die Differenz der Integrale $f_Z(P)$ und $f_A(P)$ in dem Druckintervall als Maß für die durch den Atemweg absorbierte Energie angesehen. Diese Differenz der Integrale sollte daher möglichst gering sein, so dass die durch den Atemweg absorbierte Energie als möglichst gering angesehen werden kann.

Insbesondere ist die Steuereinrichtung zur Durchführung von mehreren Beatmungsvorgängen geeignet, bei denen die Differenz von

a) $\int f_{ZP}(P)\,dP$ und $\int f_{AP}(P)\,dP$ in dem Druckintervall oder

b) $\int f_{ZV}(V)\,dV$ und $\int f_{AV}(V)\,dV$ in dem Volumenintervall

regelbar ist, wobei ein Verhältnis der Differenz zu einer für einen bestimmten Patienten festgelegten kritischen Differenz einstellbar ist.

Insbesondere kann für einen Patienten z. B. aufgrund von Erfahrungswerten oder aufgrund einer Ermittlung einer Compliance des Atemwegs eine kritische Differenz bestimmt werden. Diese kritische Differenz bezeichnet das Maß an Energie, dass während eines Beatmungsvorgangs dem zumindest einem Atemweg zugeführt werden darf ohne dass eine Schädigung (VILI) des zumindest einen Atemwegs zu erwarten ist. Die kritische Differenz kann z. B. die Differenz der Integrale von $\int f_{ZP}(P)\,dP$ und $\int f_{AP}(P)\,dP$ in dem Druckintervall sein, wobei $f_{ZP}(P)$ die Zuführung des Fluids bei größtmöglicher Compliance beschreibt und wobei $f_{AP}(P)$ die Abführung des Fluids aufgrund passiver Exspiration beschreibt (d. h. (ausschließlich) die in den elastischen Gewebeelementen von Lunge und Thorax gespeicherte Energie treibt die Exspiration an).

Insbesondere wird durch die Integrale von $\int f_{ZP}(P)\,dP$ und $\int f_{AP}(P)\,dP$ in dem Druckintervall die Fläche unterhalb des ersten Kurvenabschnitts bzw. die Fläche unterhalb des zweiten Kurvenabschnitts bestimmt (gleiches gilt für $\int f_{ZV}(V)\,dV$ und $\int f_{AV}(V)$ in dem Volumenintervall). Eine Differenz der Integrale $\int f_{ZP}(P)\,dP$ und $\int f_{AP}(P)\,dP$ in dem Druckintervall bezeichnet daher die Fläche, die von dem ersten Kurvenabschnitt und dem zweiten Kurvenabschnitt eingeschlossen wird. Die Differenz der Integrale $\int f_{ZP}(P)\,dP$ und $\int f_{AP}(P)\,dP$ in dem Druckintervall und damit die von den Kurvenabschnitten umschlossene Fläche wird als Maß für die durch den Atemweg absorbierte Energie angesehen. Diese Differenz der Integrale bzw. diese Fläche sollte daher möglichst gering sein, so dass die durch den Atemweg absorbierte Energie als möglichst gering angesehen werden kann.

Die Beatmungsvorrichtung umfasst eine Visualisierungseinrichtung und zumindest eine Fluidzuführeinheit und eine Fluidabführeinheit, die zum Zuführen eines Fluids in zumindest einen Atemweg, also in einen Lungenteil oder in die Lunge, eines Patienten sowie zum Abführen des Fluids aus diesem Atemweg geeignet ist; sowie eine Steuereinrichtung, die, zumindest während eines Beatmungsvorgangs des zumindest einen Atemwegs, also dem zumindest einmaligen Zuführen des Fluids in den zumindest einen Atemweg und dem zumindest einmaligen Abführen des Fluids aus dem zumindest einen Atemweg durch Betreiben der Beatmungsvorrichtung, zur Ermittlung eines Verlaufs zumindest einer Volumen-Druck Kurve in einem Volumen-Druck Diagramm geeignet ist. Die Kurve weist einen ersten Kurvenabschnitt $V=f_{ZP}(P)$ oder $P=f_{ZV}(V)$ und einen zweiten Kurvenabschnitt $V=f_{AP}(P)$ oder $P=f_{AV}(V)$ auf, wobei der erste Kurvenabschnitt den Verlauf des zugeführten Volumens und des Druckes während des Zuführen des Fluids in den zumindest einen Atemweg und der zweite Kurvenabschnitt den Verlauf des abgeführten Volumens und des Druckes während des Abführens des Fluids aus dem zumindest einen Atemweg darstellt; wobei der Beatmungsvorgang innerhalb eines Druckintervalls und innerhalb eines Volumenintervalls stattfindet. Die Steuereinrichtung ist zur Bestimmung einer Fläche geeignet, wobei diese Fläche in dem Volumen-Druck Diagramm von dem ersten Kurvenabschnitt und dem zweiten Kurvenabschnitt des einen Beatmungsvorgangs umschlossen ist; wobei über die Visualisierungseinrichtung zumindest einer der folgenden Parameter:

a) ein Maß für eine Größe der Fläche; oder

b) ein Maß für eine Veränderung der Fläche über mehrere Beatmungsvorgänge hinweg; oder

c) ein Maß für ein Verhältnis der Fläche zu einer für einen bestimmten Patienten festgelegten kritischen Fläche (also die kritische Differenz der Integrale); oder

d) ein Maß für eine Veränderung des Verhältnisses der Fläche zu einer für einen bestimmten Patienten festgelegten kritischen Fläche (also die kritische Differenz der Integrale) über mehrere Beatmungsvorgänge hinweg

visuell erkennbar darstellbar ist.

Insbesondere ist über die Visualisierungseinrichtung zusätzlich der Verlauf zumindest einer Volumen-Druck Kurve in einem Volumen-Druck Diagramm darstellbar. Die Kurve weist einen ersten Kurvenabschnitt $V=f_{ZP}(P)$ oder $P=f_{ZV}(V)$ und einen zweiten Kurvenabschnitt $V=f_{AP}(P)$ oder $P=f_{AV}(V)$ auf, wobei der erste Kurvenabschnitt den Verlauf des zugeführten Volumens und des Druckes während des Zuführen des Fluids in den zumindest einen Atemweg und der zweite Kurvenabschnitt den Verlauf des abgeführten Volumens und des Druckes während des Abführens des Fluids aus dem zumindest einen Atemweg darstellt.

Insbesondere umfasst die Visualisierungseinrichtung eine grafische Darstellungsfläche, z. B. einen Monitor, über den die genannten Parameter ablesbar darstellbar sind.

Insbesondere können die genannten Parameter z. B. durch eine Art "Ampeldarstellung" verdeutlicht werden. Dabei können die Werte der Parameter Bereichen zugeordnet werden, die in entsprechenden Farben darstellbar sind (z. B. grün für "unkritisch"; gelb für einen "Zwischenbereich"; rot für "kritisch").

Bevorzugt ist zumindest einer der Parameter a. bis d. in Relation zu zumindest einer Eingriffsgrenze darstellbar. "Eingriffsgrenzen" sind insbesondere bestimmte Werte der Parameter, wobei bei Erreichen der Eingriffsgrenze z. B. ein Eingreifen oder eine Regelung erforderlich sein kann. D. h. insbesondere, dass wenn ein Parameter einen bestimmten Wert erreicht, ein Hinweis über die Visualisierungseinrichtung erzeugt wird, so dass ein Bediener der Beatmungseinrichtung oder die Steuereinrichtung selber auf diesen Umstand hingewiesen wird oder ggf. eine Veränderung des Parameters bewirken kann.

Insbesondere wird die Beatmungsvorrichtung so betrieben, dass das Verfahren zumindest die folgenden Schritte umfasst:

a) Durchführen eines Beatmungsvorgangs, umfassend zumindest ein einmaliges Zuführen eines Fluids in zumindest einen Atemweg, also einen Lungenteil oder die Lunge, des Patienten und zumindest ein einmaliges Abführen des Fluids aus diesem Atemweg durch Betreiben der Beatmungsvorrichtung; wobei der Beatmungsvorgang innerhalb eines Druckintervalls und innerhalb eines Volumenintervalls stattfindet;

b) Ermitteln oder Einstellen eines Verlaufs zumindest einer Volumen-Druck Kurve in einem Volumen-Druck Diagramm durch die Steuereinrichtung während des Beatmungsvorgangs; wobei die Kurve einen ersten Kurvenabschnitt $V=f_{ZP}(P)$ oder $P=f_{ZV}(V)$ und einen zweiten Kurvenabschnitt $V=f_{AP}(P)$ oder $P=f_{AV}(V)$ aufweist, wobei der erste Kurvenabschnitt den Verlauf des zugeführten Volumens V und des Druckes P während des Zuführens des Fluids in den zumindest einen Atemweg und der zweite Kurvenabschnitt den Verlauf des abgeführten Volumens V und des Druckes P während des Abführens des Fluids aus dem zumindest einen Atemweg darstellt; wobei durch die Steuereinrichtung der Beatmungsvorgang so eingestellt wird, dass

(1) über zumindest 60 %, insbesondere zumindest 80 %, des Druckintervalls ein Verhältnis aus einem Betrag einer ersten Steigung des ersten Kurvenabschnitts bei einem Druck $P_0$, also $df_{AP}/d(P)$ ($P_0$) und einem Betrag einer zweiten Steigung des zweiten Kurvenabschnitts, also $df_{AP}/d(P)$ ($P_0$), bei demselben Druck $P_0$, einen Wert von mindestens 0,5 und höchstens 2,0, insbesondere von mindestens 0,67 und höchstens 1,5 aufweist, oder

(2) über zumindest 60 %, insbesondere zumindest 80 %, des Volumenintervalls ein Verhältnis aus einem Betrag einer ersten Steigung des ersten Kurvenabschnitts bei einem Volumen $V_0$, also $df_{AV}/d(V)$ ($V_0$) und einem Betrag einer zweiten Steigung des zweiten Kurvenabschnitts, also $df_{AV}/d(V)$ ($V_0$), bei demselben Volumen $P_0$, einen Wert von mindestens 0,5 und höchstens 2,0, insbesondere von mindestens 0,67 und höchstens 1,5 aufweist.

Insbesondere ist der Beatmungsvorgang so einstellbar, dass über zumindest 60 %, insbesondere zumindest 80 %, des Druckintervalls das Verhältnis einen Wert von mindestens 0,75 und höchstens 1,25 aufweist.

Insbesondere ermittelt die Steuereinrichtung einen Verlauf einer Volumen-Druck Kurve in einem Volumen-Druck Diagramm während des Beatmungsvorgangs für eine Compliance des Atemwegs gemäß einem von $V=f_{CP}(P)$ und $P=f_{CV}(V)$; wobei der Beatmungsvorgang in den Schritten a) und b) so eingestellt wird, dass über zumindest 60 %, insbesondere zumindest 80 % des Druckintervalls oder über zumindest 60 %, insbesondere zumindest 80 % des Volumenintervalls ein Verhältnis aus

- jedem von $df_{AP}/d(P)$ ($P_0$), $df_{ZP}/d(P)$ ($P_0$) und
- einem Betrag eines Maßes der Veränderung des bei einem Druck $P_0$ (11) vorliegenden ersten Volumens der Compliance, also $df_C/d(P)$ ($P_0$), oder

ein Verhältnis aus

- jedem von $df_{AV}/d(V)$ ($V_0$), $df_{ZV}/d(V)$ ($V_0$) und
- und einem Betrag eines Maßes der Veränderung des bei einem Volumen $V_0$ vorliegenden ersten Drucks der Compliance, also $df_{CV}/d(V)$ ($V_0$)

einen Wert von mindestens 0,5 und höchstens 2,0, insbesondere von mindestens 0,67 und höchstens 1,5, bevorzugt von mindestens 0,75 und höchstens 1,25 aufweist.

Insbesondere erfolgt in Schritt b) oder in einem weiteren Schritt c) durch die Steuereinrichtung eine Ermittlung oder eine Einstellung einer Fläche; wobei diese Fläche in dem Volumen-Druck Diagramm von dem ersten Kurvenabschnitt und dem zweiten Kurvenabschnitt des einen Beatmungsvorgangs umschlossen ist.

Bevorzugt wird bei dem zumindest einen Beatmungsvorgang ein Verhältnis der Fläche zu einer für einen bestimmten Patienten festgelegten kritischen Fläche (der kritischen Differenz der Integrale) eingestellt.

Insbesondere umfasst die Beatmungsvorrichtung eine Visualisierungseinrichtung, wobei über die Visualisierungseinrichtung zumindest einer der folgenden Parameter:

a) ein Maß für eine Größe der Fläche; oder

b) ein Maß für eine Veränderung der Fläche über mehrere Beatmungsvorgänge hinweg; oder

c) ein Maß für ein Verhältnis der Fläche zu einer für einen bestimmten Patienten festgelegten kritischen Fläche (also die kritische Differenz der Integrale); oder

d) ein Maß für eine Veränderung des Verhältnisses der Fläche zu einer für einen bestimmten Patienten festgelegten kritischen Fläche (also die kritische Differenz der Integrale) über mehrere Beatmungsvorgänge hinweg

visuell erkennbar dargestellt wird.

Insbesondere ist der Beatmungsvorgang so einstellbar, dass

a) während des Zuführens des Fluids ein bei dem Druck $P_0$ vorliegendes erstes Volumen und während des Abführens des Fluids ein bei demselben Druck $P_0$ vorliegendes zweites Volumen sich höchstens um 30 %, insbesondere um höchstens 20 %, bevorzugt um höchstens 10 %, des in dem Druckintervall vorliegenden Volumenintervalls unterscheidet oder

b) während des Zuführens des Fluids ein bei dem Volumen $V_0$ vorliegender erster Druck und während des Abführens des Fluids ein bei demselben Volumen $V_0$ vorliegender zweiter Druck sich höchstens um 30 %, insbesondere um höchstens 20 %, bevorzugt um höchstens 10 %, des in dem Volumenintervall vorliegenden Druckintervalls unterscheidet.

Insbesondere wird die vorstehend vorgeschlagene Beatmungsvorrichtung so betrieben, dass das Verfahren zumindest die folgenden Schritte umfasst:

i. Zuführen eines Fluids in zumindest einen Atemweg, also einen Lungenteil oder die Lunge, des Patienten und/oder Abführen des Fluids aus diesem Atemweg durch Betreiben der Beatmungsvorrichtung;

ii. Ermitteln oder zusätzlich Abschätzen eines Verlaufs zumindest eines Teilbereichs einer Compliance-Kurve des mindestens einen Atemwegs durch das Zuführen und/oder Abführen des Fluids in Schritt i. und Ermittlung zumindest eines Wertes der Compliance, wobei für die Compliance C gilt:

$$C = \text{delta } V \,/\, \text{delta } P \; [\text{Milliliter/Millibar}];$$

wobei die Compliance angibt, wie viel Fluid, also ein Volumen delta V [Milliliter], in den mindestens einen Atemweg eingeführt oder aus dem Atemweg entfernt wird, so dass sich ein Druck in dem Atemweg um eine Druckdifferenz delta P [Millibar] verändert;

iii. Bestimmen einer Lage eines Druckintervalls mit den Drücken P1 und P2 entlang des Verlaufs des in Schritt ii. ermittelten oder zusätzlich abgeschätzten zumindest einen Teilbereichs der Compliance-Kurve, wobei für ein in diesem Druckintervall durchgeführten Beatmungsvorgang, also eine Inspiration und/oder eine Exspiration, ein Betrag der Compliance möglichst groß ist;

iv. Zu- und/oder Abfuhr des Fluids innerhalb des in Schritt iii. ermittelten Druckintervalls in zumindest einem auf Schritt iii. folgenden Beatmungsvorgang.

Insbesondere wird dieses Verfahren mit den Schritten i. bis iv. zusätzlich und ggf. gleichzeitig oder zeitlich versetzt zu dem Verfahren mit den Schritten a) und b) durchgeführt. Dabei kann gerade bei der Zuführung des Fluids die Compliance des Atemwegs bestimmt werden und die Zuführung und Abführung des Fluids bei zumindest einem Beatmungsvorgang gemäß den Schritten a) und b) des Verfahrens erfolgen.

Insbesondere weist das Verfahren zur Beatmung eines Patienten einen möglichst geringen Energieeintrag auf. Die Minimierung des Energieeintrags wird auch durch die Ermittlung des geringstmöglichen Druckes erreicht, bei dem ein benötigtes Atemzugvolumen $V_T$ (Tidalvolumen) dem Patienten zugeführt werden kann. Diese Drücke P1 und P2 des Druckintervalls können z. B. anhand der jeweils vorliegenden Compliance des beatmeten Patienten ermittelt werden. Insbesondere wird in Schritt ii. zumindest während einer Inspiration oder einer Exspiration eines Beatmungsvorgangs eine Mehrzahl von Werten für die Compliance ermittelt, so dass in Schritt iii. für zumindest einen folgenden Beatmungsvorgang die Lage des Druckintervalls mit den Drücken P1 und P2 bestimmbar ist, für das ein Betrag der Compliance möglichst groß ist. Insbesondere werden durch die Steuereinrichtung die Werte für die Compliance kontinuierlich oder in vorbestimmbaren Zeitabständen ermittelt. Bevorzugt werden mindestens 5, besonders bevorzugt mindestens 10 Werte für die Compliance je Beatmungsvorgang bestimmt.

Bevorzugt werden die Schritte ii., iii. und iv. kontinuierlich durchgeführt, so dass wahlweise für jeden folgenden Beatmungsvorgang oder für mehrere aufeinanderfolgende Beatmungsvorgänge die Lage des Druckintervalls mit den Drücken P1 und P2 neu bestimmt wird.

Gemäß einer bevorzugten Ausgestaltung wird in Abhängigkeit von der in Schritt iii. ermittelten Lage des Druckintervalls und des Druckintervalls selbst sowie der dabei bestimmten Compliance zumindest für den folgenden Beatmungsvorgang zumindest einer der folgenden Parameter bestimmt:

- ein Atemzugvolumen $V_T$ (Tidalvolumen) [Milliliter],
- ein Druck P1 (z. B. PEEP) und ein Druck P2 (z. B. PIP) [Millibar],
- eine Beatmungsfrequenz F [1/Sekunde]
- I/E.

Gemäß einer vorteilhaften Ausgestaltung wird zumindest der Druckanstieg, also delta P / delta t [Millibar/Sekunde] während einer Inspiration kontrolliert und begrenzt.

Gemäß einer anderen vorteilhaften Ausgestaltung wird zumindest der Druckabfall, also delta P / delta t [Millibar/Sekunde] während einer Exspiration kontrolliert und begrenzt.

Bevorzugt werden der Druckanstieg und der Druckabfall kontrolliert und begrenzt.

Insbesondere wird der Betrag des Druckanstiegs oder Druckabfalls auf höchstens 40 mbar/s [Millibar/Sekunde], insbesondere höchstens 30 mbar/s, bevorzugt höchstens 20 mbar/s, besonders bevorzugt höchstens 10 mbar/s begrenzt.

Insbesondere wird für die Beatmung des Patienten ein Katheter eingesetzt, der einen, zumindest für ein während der Inspiration zugeführtes Fluid, durchströmbaren Querschnitt von höchstens 30 mm$^2$ [Quadratmillimeter], insbesondere von höchstens 20 mm$^2$ aufweist.

Insbesondere kann über einen solch geringen Querschnitt (bei ausschließlicher Inspiration und Exspiration über dieses Lumen) eine Begrenzung des Druckanstiegs während der Inspiration aber auch während der Exspiration erreicht werden.

Insbesondere kann in einer Fluidabführeinheit ein Widerstand (z. B. ein Strömungswiderstand oder ähnliches) vorgesehen sein, der den Druckabfall während der Exspiration begrenzt und kontrolliert.

Für die vorgeschlagene Beatmungsvorrichtung gilt insbesondere gleichermaßen, dass zunächst ein, für den zumindest einen Atemweg des zu beatmenden Patienten vorliegender, Teilbereich einer Compliance-Kurve ermittelt und ggf. zusätzlich abgeschätzt wird. Dafür wird der Druckanstieg während der Zugabe eines bestimmten Volumens V (z. B. 50 oder 100 ml [Milliliter]; ggf. auch $V_T$) gemessen.

Weiter wird insbesondere danach ein PEEP-Niveau bestimmt (also der kleinere Druck von P1 und P2). Zur Bestimmung des PEEP-Niveaus, mit dem der Patient im Folgenden beatmet werden soll, können zunächst auch mehrere Beatmungsvorgänge mit jeweils unterschiedlichen PEEP-Niveaus durchgeführt werden.

Weiter wird bevorzugt ein PIP-Niveau bestimmt (also der größere Druck von P1 und P2). Zur Bestimmung des PIP-Niveaus, mit dem der Patient im Folgenden beatmet werden soll, können zunächst auch mehrere Beatmungsvorgänge mit jeweils unterschiedlichen PIP-Niveaus durchgeführt werden.

Weiter wird ein für den vorliegenden Patienten erwartetes Atemzugvolumen $V_T$ eingestellt. Dieses Atemzugvolumen $V_T$ kann während der Beatmung z. B. anhand der Überwachung des $CO_2$-Niveaus weiter angepasst werden. Alternativ oder zusätzlich kann das $CO_2$-Niveau auch mittels der Frequenz der Beatmungsvorgänge oder mit der Volumenflussrate beeinflusst werden.

Insbesondere wird der Druckanstieg und/oder der Druckabfall während der Beatmung gesteuert und kontrolliert, so dass der auf den mindestens einen Atemweg einwirkende "shear-stress" und der Energieeintrag minimiert wird.

Insbesondere wird durch die Beatmungsvorrichtung sichergestellt, dass ein Betrag der Compliance während eines Beatmungsvorgangs möglichst groß ist, oder insbesondere in anderen Worten, dass

(1) die Beatmung in einem Druckintervall erfolgt, in dem das zugeführte Volumen des Fluids maximal ist, bzw.

(2) die Zu- oder Abfuhr eines vorbestimmten Volumens V bzw. eines Atemzugvolumens $V_T$ des Fluids innerhalb eines Druckintervalls erfolgt, dass möglichst klein ist.

Insbesondere wird die vorstehend beschriebene Beatmungsvorrichtung so betrieben, dass ein möglichst geringer Energieeintrag in die Atemwege des Patienten erreicht werden soll. Gemäß dem vorliegenden Verfahren erfolgt während der Beatmung des Patienten eine kontinuierliche (also zu jedem Zeitpunkt stattfindende) aktive Steuerung des während der Inspiration der Lunge des Patienten zugeführten und während der Exspiration aus der Lunge des Patienten abgeführten Fluids durch die Beatmungsvorrichtung. Die aktive Steuerung umfasst eine kontinuierliche Druckänderung des zugeführten und abgeführten Fluids durch die Beatmungsvorrichtung. Der kontinuierlich geänderte Druck ist insbesondere der Druck innerhalb der Atemwege und damit innerhalb der Lunge. Dieser Druck kann über eine Messung am Ende einer, in den Atemweg hineinreichenden Beatmungsvorrichtung, z. B. einem Katheter, über einen Sensor bestimmt werden.

Die kontinuierliche Druckänderung führt insbesondere zu einer kontinuierlichen Steuerung der Fluidzufuhrrate und Fluidabfuhrrate [Milliliter/Sekunde] durch die Beatmungsvorrichtung hin zu der Lunge bzw. aus der Lunge während der Beatmungsvorgänge. Insbesondere wird so das in der Lunge vorhandene Volumen V des Fluids kontinuierlich geändert. Bevorzugt werden während der Änderung des in der Lunge befindlichen Volumens die Fluidzufuhrrate und/oder die Fluidabfuhrrate durch die Beatmungsvorrichtung zu der Lunge hin bzw. aus der Lunge nicht verändert und bleibt entsprechend im Wesentlichen konstant. Dabei muss die Fluidzufuhrrate nicht zwingend der Fluidabfuhrrate entsprechen, sie kann aber auch betragsgleich sein. Weiter kann die Fluidzufuhrrate von einem Inspirationsvorgang zum folgenden Inspirationsvorgang variiert werden. Gleiches gilt - insbesondere davon unabhängig - für die Fluidabfuhrrate während aufeinanderfolgender Exspirationsvorgänge.

Insbesondere werden Zustände, in denen innerhalb eines Zeitintervalls keine Änderung des Drucks und insbesondere keine Änderung des in der Lunge vorhandenen Fluidvolumens erfolgen, vermieden. Bevorzugt sind solche Zeitintervalle, in denen keine Änderung des Drucks und/oder insbesondere keine Änderung des in der Lunge vorhandenen Fluidvolumens erfolgen, höchstens 0,5 s [Sekunden], insbesondere höchstens 0,2 s, bevorzugt höchstens 0,1 s lang und betreffen insbesondere (ausschließlich) den Zeitpunkt der Fluidstromumkehr (also Übergang von Fluidzufuhr zu Fluidabfuhr und umgekehrt).

Die Beatmungsvorrichtung umfasst zumindest eine Fluidzuführeinheit und eine Fluidabführeinheit, die zum Zuführen eines Fluids in zumindest einen Atemweg, also in einen Lungenteil oder in die Lunge, eines Patienten sowie zum Abführen des Fluids aus diesem Atemweg geeignet ist; sowie eine Steuereinrichtung, die, zumindest während eines Beatmungsvorgangs des zumindest einen Atemwegs, also dem zumindest einmaligen Zuführen des Fluids in den zumindest einen Atemweg und dem zumindest einmaligen Abführen des Fluids aus dem zumindest einen Atemweg durch Betreiben der Beatmungsvorrichtung, zur Einstellung eines Verlaufs eines Druckes P in dem Atemweg und eines Verlaufs eines dem Atemweg zugeführten und eines aus dem Atemweg abgeführten Volumens V des Fluids gemäß $V=f_{ZP}(P)$ und $V=f_{AP}(P)$ oder gemäß $P=f_{ZV}(V)$ und $P=f_{AV}(V)$ eingerichtet ist. Der Beatmungsvorgang findet innerhalb eines Druckintervalls und innerhalb eines Volumenintervalls statt; wobei durch die Steuereinrichtung der Beatmungsvorgang so einstellbar ist, dass während des Zuführens des Fluids und während des Abführens des Fluids eine (ggf. über die Zeit variierende) Volumenflussrate F(t) [l/min] gegenüber einer, in dem Beatmungsvorgang durchschnittlichen Volumenflussrate FD, zumindest während 80 %, bevorzugt während 90 % der Dauer des Beatmungsvorgangs, um höchstens 50 %, insbesondere um höchstens 25 % variiert.

Die Volumenflussrate F(t) kann insbesondere über die Zeit variieren, wobei jedoch gerade eine (möglichst) konstante Volumenflussrate F(t) (vom Betrag her) eingestellt werden sollte. Die durchschnittliche Volumenflussrate FD wird ermittelt, in dem die Summe aus zugeführten und abgeführten Fluid (also immer ein positiver Wert) durch die Dauer des Beatmungsvorgangs dividiert wird. Die durchschnittliche Volumenflussrate kann für die Einstellung und Steuerung der Volumenflussrate F(t) auch auf Basis der vorherigen Beatmungsvorgänge oder auf Basis der voreingestellten Parameter (z. B. Frequenz und Tidalvolumen) ermittelt werden.

Es wurde im Rahmen der vorliegenden Erfindung festgestellt, dass gerade auch ein Unterschied in der Volumenflussrate F(t) die von dem Atemweg absorbierte Energie beeinflusst.

Die Beatmungsvorrichtung umfasst eine Visualisierungseinrichtung und zumindest eine Fluidzuführeinheit und eine Fluidabführeinheit, die zum Zuführen eines Fluids in zumindest einen Atemweg, also in einen Lungenteil oder in die Lunge, eines Patienten sowie zum Abführen des Fluids aus diesem Atemweg geeignet ist; sowie eine Steuereinrichtung, die, zumindest während eines Beatmungsvorgangs des zumindest einen Atemwegs, also dem zumindest einmaligen Zuführen des Fluids in den zumindest einen Atemweg und dem zumindest einmaligen Abführen des Fluids aus dem zumindest einen Atemweg durch Betreiben der Beatmungsvorrichtung, zur Ermittlung und Einstellung einer Volumenflussrate F(t) [l/min] des Fluids geeignet ist. Der Beatmungsvorgang findet innerhalb eines Druckintervalls und innerhalb eines Volumenintervalls statt; wobei die Steuereinrichtung unter Annahme eines Atemwegswiderstands R des Atemwegs des Patienten zur Bestimmung einer Verlustleistung PW(t) [Watt] des Atemwegs gemäß $PW(t) = R_1 * (F(t))^3 + R_2 * (F(t))^2$, mit $R_1 = R$ [Pascals/(m³/s)²] und $R_2 = R$ [Pascals/(m³/s)] (wobei die Einheit [Pascals/(Kubikmeter/Sekunde) bzw. [Pascals/(Kubikmeter/Sekunde)²] lautet) geeignet ist; wobei über die Visualisierungseinrichtung zumindest einer der folgenden Parameter:

a) die Verlustleistung PW(t); oder
b) eine Verlustenergie E [Joule], nämlich das Integral von PW(t)dt, also JPW(t)dt in einem Zeitintervall; oder
c) ein Maß für ein Verhältnis der Verlustleistung PW(t) zu einer für einen bestimmten Patienten festgelegten kritischen Verlustleistung; oder
d) ein Maß für ein Verhältnis der Verlustenergie E zu einer für einen bestimmten Patienten festgelegten kritischen Verlustenergie

visuell erkennbar darstellbar ist.

Die hier angeführte Verlustenergie bezeichnet den eingangs erwähnten Energieeintrag in den Atemweg. Diesen gilt es zu minimieren. Der Atemwegwiderstand R kann, wie vorstehend bereits ausgeführt, z. B. durch eine Plethysmographie ermittelt werden.

Insbesondere umfasst die Visualisierungseinrichtung eine grafische Darstellungsfläche, z. B. einen Monitor, über den die genannten Parameter ablesbar darstellbar sind.

Insbesondere können die genannten Parameter z. B. durch eine Art "Ampeldarstellung" verdeutlicht werden. Dabei können die Werte der Parameter Bereichen zugeordnet werden, die in entsprechenden Farben darstellbar sind (z. B. grün für "unkritisch"; gelb für einen "Zwischenbereich"; rot für "kritisch").

Bevorzugt ist zumindest einer der Parameter a. bis d. in Relation zu zumindest einer Eingriffsgrenze darstellbar. "Eingriffsgrenzen" sind insbesondere bestimmte Werte der Parameter, wobei bei Erreichen der Eingriffsgrenze z. B. ein Eingreifen oder eine Regelung erforderlich sein kann. D. h. insbesondere, dass wenn ein Parameter einen bestimmten Wert erreicht, ein Hinweis über die Visualisierungseinrichtung erzeugt wird, so dass ein Bediener der Beatmungseinrichtung oder die Steuereinrichtung selber auf diesen Umstand hingewiesen wird oder ggf. eine Veränderung des Parameters bewirken kann.

Insbesondere wird die Beatmungsvorrichtung so betrieben, dass das Verfahren zumindest die folgenden Schritte umfasst:

a) Durchführen eines Beatmungsvorgangs, umfassend zumindest ein einmaliges Zuführen eines Fluids in zumindest einen Atemweg, also einen Lungenteil oder die Lunge, des Patienten und zumindest ein einmaliges Abführen des Fluids aus diesem Atemweg durch Betreiben der Beatmungsvorrichtung; wobei der Beatmungsvorgang innerhalb eines Druckintervalls und innerhalb eines Volumenintervalls stattfindet;

b) Ermitteln oder Einstellen eines Verlaufs eines Druckes P in dem Atemweg und eines Verlaufs eines dem Atemweg zugeführten und eines aus dem Atemweg abgeführten Volumens V des Fluids gemäß $V=f_{ZP}(P)$ und $V=f_{AP}(P)$ oder gemäß $P= f_{ZV}(V)$ und $P=f_{AV}(V)$; wobei durch die Steuereinrichtung der Beatmungsvorgang so eingestellt wird, dass während des Zuführen des Fluids und während des Abführens des Fluids eine Volumenflussrate F(t) [l/min] gegenüber einer, in dem Beatmungsvorgang durchschnittlichen Volumenflussrate FD zumindest während 80 %, bevorzugt während 90 % der Dauer des Beatmungsvorgangs, um höchstens 50 %, insbesondere um höchstens 25 % variiert.

Insbesondere umfasst die Beatmungsvorrichtung eine Visualisierungseinrichtung, wobei über die Visualisierungseinrichtung zumindest einer der folgenden Parameter:

a. die Verlustleistung PW(t); oder

b. eine Verlustenergie E [Joule], nämlich das Integral von PW(t)dt, also ∫PW(t)dt in einem Zeitintervall; oder

c. ein Maß für ein Verhältnis der Verlustleistung PW(t) zu einer für einen bestimmten Patienten festgelegten kritischen Verlustleistung; oder

d. ein Maß für ein Verhältnis der Verlustenergie E zu einer für einen bestimmten Patienten festgelegten kritischen Verlustenergie

visuell erkennbar dargestellt wird.

Die Beatmungsvorrichtung umfasst zumindest eine Fluidzuführeinheit und eine Fluidabführeinheit, die zum Zuführen eines Fluids in zumindest einen Atemweg, also in einen Lungenteil oder in die Lunge, eines Patienten sowie zum Abführen des Fluids aus diesem Atemweg geeignet ist; sowie eine Steuereinrichtung, die, zumindest während eines Beatmungsvorgangs des zumindest einen Atemwegs, also dem zumindest einmaligen Zuführen des Fluids in den zumindest einen Atemweg und dem zumindest einmaligen Abführen des Fluids aus dem zumindest einen Atemweg durch Betreiben der Beatmungsvorrichtung, zur Einstellung eines Verlaufs eines Druckes P in dem Atemweg und eines Verlaufs eines dem Atemweg zugeführten und eines aus dem Atemweg abgeführten Volumens V des Fluids gemäß $V=f_{ZP}(P)$ und $V=f_{AP}(P)$ oder gemäß $P=f_{ZV}(V)$ und $P=f_{AV}(V)$ eingerichtet ist, wobei der Beatmungsvorgang innerhalb eines Druckintervalls und innerhalb eines Volumenintervalls stattfindet. Durch die Steuereinrichtung ist der Beatmungsvorgang so einstellbar, dass ein Quadrat einer Geschwindigkeit $(s(t))^2$ des Verlaufs des Druckes P [cmH₂O] und des Volumens V [ml] während des Zuführens des Fluids und während des Abführen des Fluids, also $(s(t))^2 = (dP/dt)^2 + (dV/dt)^2$, gegenüber einem während des Beatmungsvorgangs durchschnittlichen Quadrat einer Geschwindigkeit $sD^2$ zumindest während 80 %, bevorzugt während 90 % der Dauer des Beatmungsvorgangs, um höchstens 300 %, bevorzugt höchstens 200 % variiert.

Im Rahmen der vorliegenden Erfindung wurde erkannt, dass die Verlustleistung P(t) sich proportional zu dem hier angeführten Quadrat einer Geschwindigkeit $(s(t))^2$ des Verlaufs des Druckes P [cmH₂O] und des Volumens V [ml] während des Zuführens des Fluids und während des Abführen des Fluids verhält.

Aus diesem Grund wird hier das Quadrat einer Geschwindigkeit $(s(t))^2$ ermittelt, dass insbesondere über eine Steuerung der Beatmungsvorrichtung errechenbar ist.

Das Quadrat einer Geschwindigkeit $(s(t))^2$ kann insbesondere über die Zeit variieren, wobei jedoch gerade eine (möglichst) konstante Geschwindigkeit s(t) eingestellt werden sollte. Das durchschnittliche Quadrat einer Geschwindigkeit $sD^2$ wird ermittelt gemäß $sD^2 = (delta P/t)^2 + (delta V/t)^2$, wobei delta P das Druckintervall des Beatmungsvorgangs, delta V das Volumenintervall des Beatmungsvorgangs und t die Dauer des Beatmungsvorgangs ist. Das durchschnittliche Quadrat einer Geschwindigkeit kann für die Einstellung und Steuerung des aktuellen Beatmungsvorgangs auch auf Basis der vorherigen Beatmungsvorgänge oder auf Basis der voreingestellten Parameter (z. B. Volumenflussrate, PEEP und PIP sowie $V_T$ und Frequenz) ermittelt werden.

Die Beatmungsvorrichtung umfasst eine Visualisierungseinrichtung und zumindest eine Fluidzuführeinheit und eine Fluidabführeinheit, die zum Zuführen eines Fluids in zumindest einen Atemweg, also in einen Lungenteil oder in die Lunge, eines Patienten sowie zum Abführen des Fluids aus diesem Atemweg geeignet ist; sowie eine Steuereinrichtung, die, zumindest während eines Beatmungsvorgangs des zumindest einen Atemwegs, also dem zumindest einmaligen Zuführen des Fluids in den zumindest einen Atemweg und dem zumindest einmaligen Abführen des Fluids aus dem zumindest einen Atemweg durch Betreiben der Beatmungsvorrichtung, zur Ermittlung eines Verlaufs zumindest einer Volumen-Druck Kurve in einem Volumen-Druck Diagramm geeignet ist; wobei die Kurve einen ersten Kurvenabschnitt, $V=f_{ZP}(P)$ oder $P=f_{ZV}(V)$, und einen zweiten Kurvenabschnitt, $V=f_{AP}(P)$ oder $P=f_{AV}(V)$, aufweist, wobei der erste Kurvenabschnitt den Verlauf des zugeführten Volumens V und des Druckes P während des Zuführen des Fluids in den zumindest einen Atemweg und der zweite Kurvenabschnitt den Verlauf des abgeführten Volumens V und des Druckes P während des Abführens des Fluids aus dem zumindest einen Atemweg darstellt; wobei der Beatmungsvorgang innerhalb eines Druckintervalls und innerhalb eines Volumenintervalls stattfindet. Die Steuereinrichtung ist zur Bestimmung eines Quadrats einer Geschwindigkeit $(s(t))^2$ des Verlaufs des Druckes P [cmH₂O] und des Volumens V [ml] während des Zuführens des Fluids und während des Abführen des Fluids, also $(s(t))^2 = (dP/dt)^2 + (dV/dt)^2$ geeignet. Über die Visualisierungseinrichtung ist zumindest einer der folgenden Parameter:

a) das Quadrat der Geschwindigkeit s(t), also $(s(t))^2$ oder

b) das Integral von $(s(t))^2$dt, also $\int (s(t))^2$dt in einem Zeitintervall; oder

c) ein Maß für ein Verhältnis des Quadrats der Geschwindigkeit $(s(t))^2$ zu einer für einen bestimmten Patienten festgelegten kritischen Geschwindigkeit zum Quadrat; oder

d) ein Maß für ein Verhältnis des Integrals von $(s(t))^2$dt zu einem für einen bestimmten Patienten festgelegten kritischen Wert dieser Größe

visuell erkennbar darstellbar.

Insbesondere umfasst die Visualisierungseinrichtung eine grafische Darstellungsfläche, z. B. einen Monitor, über den die genannten Parameter ablesbar darstellbar sind.

Insbesondere können die genannten Parameter z. B. durch eine Art "Ampeldarstellung" verdeutlicht werden. Dabei können die Werte der Parameter Bereichen zugeordnet werden, die in entsprechenden Farben darstellbar sind (z. B. grün für "unkritisch"; gelb für einen "Zwischenbereich"; rot für "kritisch").

Bevorzugt ist zumindest einer der Parameter a. bis d. in Relation zu zumindest einer Eingriffsgrenze darstellbar. "Eingriffsgrenzen" sind insbesondere bestimmte Werte der Parameter, wobei bei Erreichen der Eingriffsgrenze z. B. ein Eingreifen oder eine Regelung erforderlich sein kann. D. h. insbesondere, dass wenn ein Parameter einen bestimmten Wert erreicht, ein Hinweis über die Visualisierungseinrichtung erzeugt wird, so dass ein Bediener der Beatmungseinrichtung oder die Steuereinrichtung selber auf diesen Umstand hingewiesen wird oder ggf. eine Veränderung des Parameters bewirken kann.

Die Beatmungsvorrichtung wird insbesondere so betrieben, dassdas Verfahren zumindest die folgenden Schritte umfasst:

a) Durchführen eines Beatmungsvorgangs, umfassend zumindest ein einmaliges Zuführen eines Fluids in zumindest einen Atemweg, also einen Lungenteil oder die Lunge, des Patienten und zumindest ein einmaliges Abführen des Fluids aus diesem Atemweg durch Betreiben der Beatmungsvorrichtung; wobei der Beatmungsvorgang innerhalb eines Druckintervalls und innerhalb eines Volumenintervalls stattfindet;

b) Ermitteln oder Einstellen eines Verlaufs zumindest einer Volumen-Druck Kurve in einem Volumen-Druck Diagramm durch die Steuereinrichtung während des Beatmungsvorgangs; wobei die Kurve einen ersten Kurvenabschnitt, $V=f_{ZP}(P)$ oder $P=f_{ZV}(V)$, und einen zweiten Kurvenabschnitt, $V=f_{AP}(P)$ oder $P=f_{AV}(V)$, aufweist, wobei der erste Kurvenabschnitt den Verlauf des zugeführten Volumens V und des Druckes P während des Zuführen des Fluids in den zumindest einen Atemweg und der zweite Kurvenabschnitt den Verlauf des abgeführten Volumens V und des Druckes P während des Abführens des Fluids aus dem zumindest einen Atemweg darstellt; wobei durch die Steuereinrichtung der Beatmungsvorgang so eingestellt wird, dass ein Quadrat einer Geschwindigkeit $(s(t))^2$ des Verlaufs des Druckes P [$cmH_2O$] und des Volumens V [ml] während des Zuführens des Fluids und während des Abführen des Fluids, also $(s(t))^2 = (dP/dt)^2 + (dV/dt)^2$, gegenüber einem während des Beatmungsvorgangs durchschnittlichen Quadrat einer Geschwindigkeit $sD^2$ zumindest während 80 %, bevorzugt während 90 % der Dauer des Beatmungsvorgangs, um höchstens 300 %, bevorzugt höchstens 200 % variiert.

Insbesondere umfasst die Beatmungsvorrichtung eine Visualisierungseinrichtung, wobei über die Visualisierungseinrichtung zumindest einer der folgenden Parameter:

a) das Quadrat der Geschwindigkeit s(t), also $(s(t))^2$ oder

b) das Integral von $(s(t))^2$dt, also $\int(s(t))^2$dt in einem Zeitintervall; oder

c) ein Maß für ein Verhältnis des Quadrats der Geschwindigkeit zu einer für einen bestimmten Patienten festgelegten kritischen Geschwindigkeit zum Quadrat (33); oder

d) ein Maß für ein Verhältnis des Integrals von $(s(t))^2$dt zu einem für einen bestimmten Patienten festgelegten kritischen Wert dieser Größe (34).

visuell erkennbar dargestellt wird.

Die Ausführungen zu der Beatmungsvorrichtungeund den Verfahren zum Betrieb der Beatmungsvorrichtung sind auf die jeweils anderen Gegenstände übertragbar. Insbesondere sind bei der Beatmungsvorrichtung und dem Verfahren die genannten Parameter und Bedingungen miteinander kombinierbar. Insbesondere bilden zumindest einzelne (oder sogar alle) der genannten Parameter und Bedingungen (gemeinsame) Randbedingungen für einen Beatmungsvorgang (oder ein Beatmungsverfahren, das sich über mehrere aufeinanderfolgende Beatmungsvorgänge erstreckt). Insbesondere sollte ein Beatmungsvorgang also so durchgeführt werden, dass er allen in den unterschiedlichen Beatmungsvorrichtungen bzw. Verfahren beschriebenen Ausführungsformen entspricht.

Es wird ausdrücklich darauf hingewiesen, dass die Steuereinrichtung auch unabhängig von der Beatmungsvorrichtung beansprucht werden kann. Die Steuereinrichtung dient insbesondere der Regelung der Beatmungsvorgänge. Hierdurch wird festgelegt, anhand welcher Größen der Beatmungsvorgang gesteuert wird und welche Parameter (maximaler/minimaler Druck, maximale/minimale Volumenflussrate, Steigung des ersten und zweiten Kurvenabschnitts, Fläche, Differenz der Flächen, etc.) dabei überwacht werden.

Vorsorglich sei angemerkt, dass die hier verwendeten Zahlwörter ("erste", "zweite", "dritte",...) vorrangig (nur) zur Unterscheidung von mehreren gleichartigen Gegenständen, Größen oder Prozessen dienen, also insbesondere keine Abhängigkeit und/oder Reihenfolge dieser Gegenstände, Größen oder Prozesse zueinander zwingend vorgeben. Sollte eine Abhängigkeit und/oder Reihenfolge erforderlich sein, ist dies hier explizit angegeben oder es ergibt sich offensichtlich für den Fachmann beim Studium der konkret beschriebenen Ausgestaltung.

Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen, diese jedoch nicht darauf beschränkt ist. Dabei sind gleiche Bauteile in den Figuren mit denselben Bezugszeichen versehen. Es zeigen schematisch:

Fig. 1:      eine Beatmungsvorrichtung und einen Patienten;

Fig. 2:      einen Verlauf einer Compliance-Kurve;

Fig. 3:      eine erste Darstellung eines Beatmungsvorgangs in einem Volumen-Druck Diagramm;

Fig. 4:      eine zweite Darstellung eines Beatmungsvorgangs in einem Volumen-Druck Diagramm;

Fig. 5:      ein erstes Diagramm, in dem eine Volumenflussrate über der Zeit dargestellt ist;

Fig. 6:      ein zweites Diagramm, in dem eine Volumenflussrate über der Zeit dargestellt ist;

Fig. 7:      ein drittes Diagramm, in dem ein Quadrat einer Geschwindigkeit über der Zeit dargestellt ist; und

Fig. 8:      ein viertes Diagramm, in dem ein Quadrat einer Geschwindigkeit über der Zeit dargestellt ist.

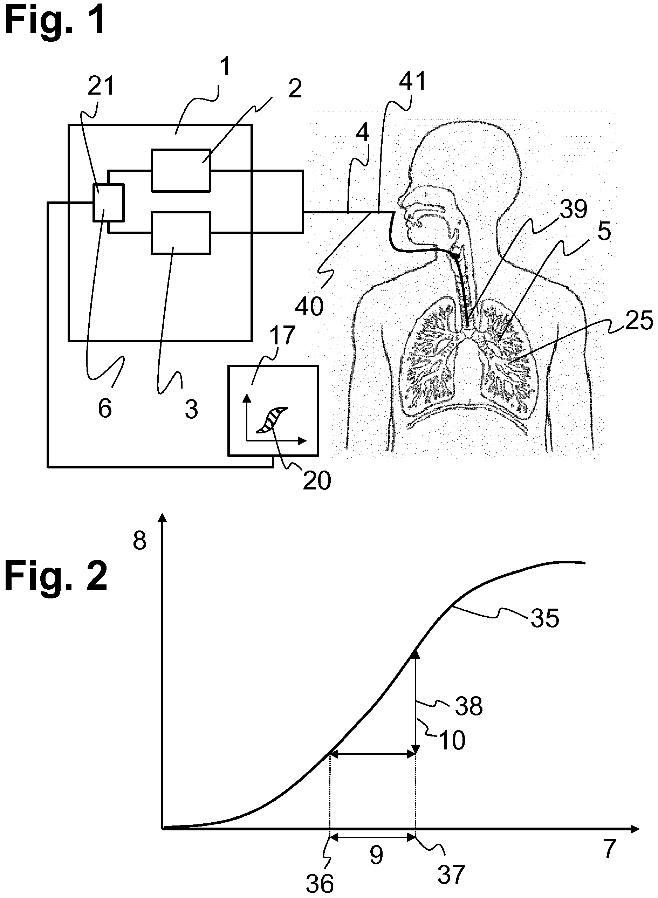

Fig. 1 zeigt eine Beatmungsvorrichtung 1 und einen Patienten mit zumindest einem Atemweg 5, bzw. einer Lunge. Die Beatmungsvorrichtung 1 umfasst eine Fluidzuführeinheit 2 und eine Fluidabführeinheit 3, die zum Zuführen eines Fluids 4 in einen Atemweg 5, also in einen Lungenteil oder in die Lunge, eines Patienten und zum Abführen des Fluids 4 aus diesem Atemweg 5 geeignet sind. Weiter umfasst die Beatmungsvorrichtung 1 eine Steuereinrichtung 6, die, während einer Beatmung des zumindest einen Atemwegs 5 des Patienten, also dem Zuführen eines Fluids 4 in den zumindest einen Atemweg 5 und dem Abführen des Fluids 4 aus dem zumindest einen Atemweg 5 durch Betreiben der Beatmungsvorrichtung 1, zur Einstellung eines Verlaufs eines Druckes P 7 in dem Atemweg 5 und eines Verlaufs eines dem Atemweg 5 zugeführten und eines aus dem Atemweg 5 abgeführten Volumens V 8 des Fluids 4 gemäß $V = f_{ZP}(P)$ und $V = f_{AP}(P)$ eingerichtet ist. Die Beatmungsvorrichtung 1 ist über einen Katheter 40 des Lumens, der einen von dem Fluid 4 durchströmbaren Querschnitt 41 aufweist, mit dem Atemweg 5 des Patienten verbunden. Die Beatmung erfolgt also z. B. über ein einziges Lumen, insbesondere unter Verwendung einer Gaststromumkehr-Vorrichtung.

Die Beatmungsvorrichtung 1 weist eine Visualisierungseinrichtung 17 auf, wobei über die Visualisierungseinrichtung 17 zumindest einer der folgenden Parameter: ein Maß für eine Größe der Fläche 20; oder ein Maß für eine Veränderung der Fläche 20 über mehrere Beatmungsvorgänge hinweg; oder ein Maß für ein Verhältnis der Fläche 20 zu einer für einen bestimmten Patienten festgelegten kritischen Fläche 21; oder ein Maß für eine Veränderung des Verhältnisses der Fläche 20 zu einer für einen bestimmten Patienten festgelegten kritischen Fläche 21 über mehrere Beatmungsvorgänge hinweg visuell erkennbar darstellbar ist.

Ein Drucksensor 39 ist innerhalb des Atemwegs 5 an dem Katheter 40 angeordnet. Der Atemweg 5 weist eine Compliance C 25 auf.

Fig. 2 zeigt einen Verlauf einer Compliance-Kurve 35 in einem Druck-Volumen Diagramm. An der horizontalen Achse ist der Druck 7 aufgetragen, an der vertikalen Achse das Volumen 8. Der Verlauf der Compliance-Kurve 35 ist für jeden Patienten individuell zu ermitteln. Weiter kann sich der Verlauf auch während einer Beatmung verändern.

Im Rahmen des Verfahrens bzw. durch die Beatmungsvorrichtung 1 wird zunächst mindestens ein Wert der Compliance 25 ermittelt, wobei für die Compliance C 25 gilt: C = delta Volumen V 8 / delta P 7 in Milliliter/Millibar. In dem hier gezeigten Teilbereich der Compliance-Kurve 35 ist der Betrag der Compliance 25 maximal. Durch Ermittlung bzw. Abschätzen des Verlaufs der Compliance-Kurve 25 kann nun die Lage eines Druckintervalls 9 mit den Drücken P1 36 und P2 37 bestimmt werden, in dem ein Atemzugvolumen $V_T$ 38 des Fluids 4 dem mindestens einen Atemweg 5 zugeführt werden kann. Diese Drücke P1 36 und P2 37 werden an der Beatmungsvorrichtung 1 eingestellt, so dass zumindest ein Beatmungsvorgang, also eine Inspiration und/oder eine Exspiration, jeweils mit einem Atemzugvolumen $V_T$ 38, zwischen diesen Drücken P1 36 und P2 37 erfolgt.

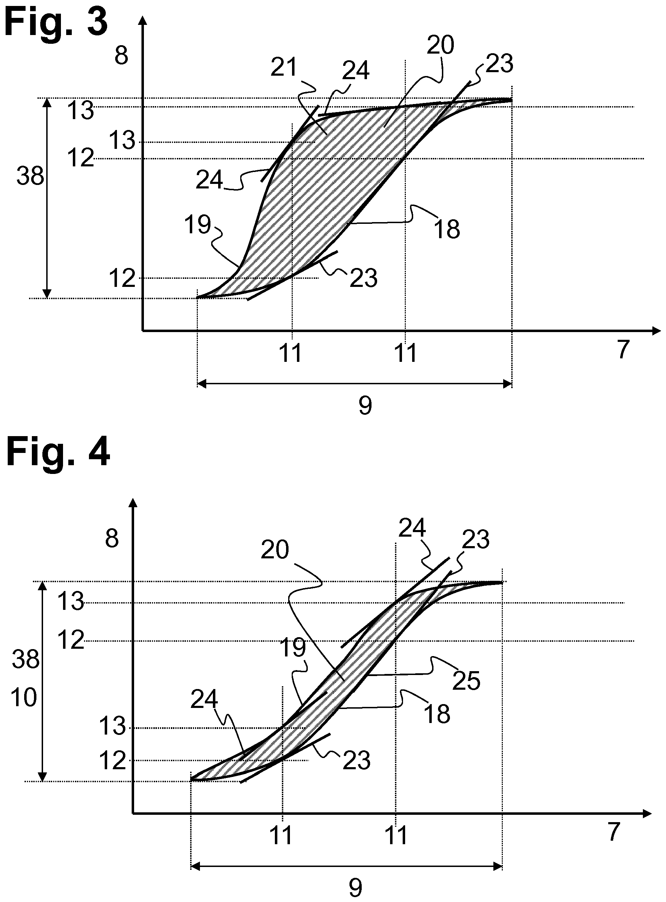

Fig. 3 zeigt eine erste Darstellung eines Beatmungsvorgangs in einem Volumen-Druck Diagramm. An der horizontalen Achse ist der Druck 7 aufgetragen, an der vertikalen Achse das Volumen 8. Die gezeigte Volumen-Druck Kurve zeigt den Verlauf des Druckes P 7 in dem Atemweg 5 während sich das Volumen V 8 in dem Atemweg 5 um das Atemzugvolumen $V_T$ 38, also einerseits um das zugeführte Volumen V 8 an Fluid 4 bzw. andererseits um das abgeführte Volumen V 8 an Fluid 4 verändert. Dabei weist die Kurve einen ersten Kurvenabschnitt 18 $V = f_{ZP}(P)$ (die untere Kurve, die sich von einem kleinsten Druck 7 und einem kleinsten Volumen 8 hin zu einem größten Druck 7 und einem größten Volumen 8 erstreckt) und einen zweiten Kurvenabschnitt 19 $V = f_{AP}(P)$ (die obere Kurve, die sich im Anschluss an den ersten Kurvenabschnitt 18 von einem größten Druck 7 und einem größten Volumen 8 hin zu einem kleinsten Druck 7 und einem kleinsten Volumen 8 erstreckt) auf, wobei der erste Kurvenabschnitt 18 den Verlauf des zugeführten Volumens V 8 (Atemzugvolumen $V_T$ 38) und des Druckes P 7 während des Zuführens des Fluids 4 in den zumindest einen Atemweg 5 und der zweite Kurvenabschnitt 19 den Verlauf des abgeführten Volumens V 8 (Atemzugvolumen $V_T$ 38) und des Druckes P 7 während des Abführens des Fluids 4 aus dem zumindest einen Atemweg 5 darstellt.

Das Maß der Veränderung des bei einem Druck $P_0$ 11 vorliegenden ersten Volumens 12 ist z. B. die erste Steigung 23 der Volumen-Druck Kurve, also hier des ersten Kurvenabschnitts 18, in einem Volumen-Druck Diagramm. Die Steigung (erste Steigung 23 und zweite Steigung 24) wird durch die erste Ableitung der jeweiligen Funktion $V=f_{ZP}(P)$ und $V=f_{AP}(P)$ ermittelt, also $df_{AP}/d(P)$ $(P_0)$ und $df_{ZP}/d(P)$ $(P_0)$.

Es wurde nun festgestellt, dass die erste Steigung 23 eines ersten Kurvenabschnitts 18 bei einem Druck $P_0$ 11 einer Volumen-Druck Kurve, darstellbar in einem Volumen-Druck Diagramm, und die zweite Steigung 24 eines zweiten Kurvenabschnitts 19 (also der Betrag eines Maßes der Veränderung eines bei demselben Druck $P_0$ 11 vorliegenden zweiten Volumens 13 während des Abführens des Fluids 4), bei einem jeweils gleichen Druck $P_0$ 11 (der Druck $P_0$ 11 liegt innerhalb des Druckintervalls 9) in einem möglichst großen Bereich des Druckintervalls 9 möglichst gleich groß sein sollte.

Dabei wird durch die Steuereinrichtung 6 zum einen der Druck- und der Volumenverlauf während der Zuführung von Fluid 4 in den zumindest einen Atemweg 5 gesteuert und kontrolliert. In Abhängigkeit von dem Verlauf dieses ersten Kurvenabschnitts 18 wird nun aber auch die Abführung des Fluids 4 aus dem Atemweg 5 gesteuert und kontrolliert. Hier wird nun insbesondere keine passive Exspiration (siehe Fig. 3) zugelassen, die üblicherweise einen von dem ersten Kurvenabschnitt 18 stark abweichenden zweiten Kurvenabschnitt 19 und damit eine große Fläche 20 erzeugen würde. Im Gegenteil, es wird vorgeschlagen auch die Abführung des Fluids 4 aktiv durch die Steuereinrichtung 6 zu kontrollieren und zu steuern, wobei der zweite Kurvenabschnitt 19 dem Verlauf des ersten Kurvenabschnitts 18 angenähert wird (siehe Fig. 4).

Erste Versuche mit derartigen Beatmungsvorrichtungen und Verfahren haben ergeben, dass durch eine derartige Steuerung der Beatmung eine Schädigung des zumindest einen Atemwegs 5 (VILI- ventilator induced lung injury) zumindest verringert oder sogar effektiv verhindert werden kann.

Die Steuereinrichtung 6 ist dafür zur Bestimmung von Integralen von $f_{ZP}(P)$ und $f_{AP}(P)$ in dem Druckintervall 9 sowie zur Bestimmung einer Differenz von $\int f_{ZP}(P)\,dP$ und $\int f_{AP}(P)\,dP$ in dem Druckintervall 9 geeignet.

Die Steuereinrichtung 6 ist zur Durchführung von mehreren Beatmungsvorgängen geeignet, bei denen die Differenz von $\int f_{ZP}(P)\,dP$ und $\int f_{AP}(P)\,dP$ in dem Druckintervall 9 regelbar ist, wobei ein Verhältnis der Differenz zu einer für einen bestimmten Patienten festgelegten kritischen Differenz einstellbar ist.

Dabei wird durch das Integral von $f_{ZP}(P)$ und $f_{AP}(P)$, also $\int f_{ZP}(P)\,dP$ und $\int f_{AP}(P)\,dP$, in dem Druckintervall 9 die Fläche unterhalb des ersten Kurvenabschnitts 18 bzw. die Fläche unterhalb des zweiten Kurvenabschnitts 19 bestimmt. Eine Differenz der Integrale $f_{ZP}(P)$ und $f_{AP}(P)$ in dem Druckintervall 9 bezeichnet daher die Fläche 20, die von dem ersten Kurvenabschnitt 18 und dem zweiten Kurvenabschnitt 19 eingeschlossen wird. Die Differenz der Integrale $f_{ZP}(P)$ und $f_{AP}(P)$ in dem Druckintervall 9 und damit die von den Kurvenabschnitten 18, 19 umschlossene Fläche 20 wird als Maß für die durch den Atemweg 5 absorbierte Energie E angesehen. Diese Differenz der Integrale bzw. diese Fläche 20 sollte daher möglichst gering sein, so dass die durch den Atemweg 5 absorbierte Energie E möglichst gering ist. Einen derartig eingestellten Beatmungsvorgang zeigt Fig. 4.

Insbesondere kann für einen Patienten z. B. aufgrund von Erfahrungswerten oder aufgrund einer Ermittlung einer Compliance 25 des Atemwegs 5 eine kritische Differenz, also eine kritische Fläche 21, bestimmt werden. Diese kritische Differenz bzw. kritische Fläche 21 bezeichnet das Maß an Energie E, das während eines Beatmungsvorgangs dem zumindest einem Atemweg 5 zugeführt werden darf, ohne dass eine Schädigung (VILI) des zumindest einen Atemwegs 5 zu erwarten ist. Die kritische Differenz kann z. B. die Differenz der Integrale von $f_{ZP}(P)$ und $f_{AP}(P)$ in dem Druckintervall 9 sein, wobei $f_{ZP}(P)$, also der erste Kurvenabschnitt 18 im Diagramm gemäß Fig. 3, die Zuführung des Fluids 4 bei größtmöglicher Compliance 25 beschreibt und wobei $f_{AP}(P)$, also der zweite Kurvenabschnitt 19 im Diagramm gemäß Fig. 3, die Abführung des Fluids 4 aufgrund passiver Exspiration beschreibt (d. h. (ausschließlich) die in den elastischen Gewebeelementen von Lunge und Thorax gespeicherte Energie treibt die Exspiration an). Die kritische Differenz ist in Fig. 3 also die kritische Fläche 21 zwischen den Kurvenabschnitten 18, 19.

Fig. 4 zeigt eine zweite Darstellung eines Beatmungsvorgangs in einem Volumen-Druck Diagramm. Auf die Ausführungen zu Fig. 3 wird verwiesen.

Die Steuereinrichtung 6 ist, zumindest während des einen gezeigten Beatmungsvorgangs des zumindest einen Atemwegs 5, also dem zumindest einmaligen Zuführen des Fluids 4 in den zumindest einen Atemweg 5 und dem zumindest einmaligen Abführen des Fluids 4 aus dem zumindest einen Atemweg 5 durch Betreiben der Beatmungsvorrichtung 1, zur Einstellung eines Verlauf eines Druckes P 7 in dem Atemweg 5 und eines Verlaufs eines dem Atemweg 5 zugeführten und eines aus dem Atemweg 5 abgeführten Volumens V 8 des Fluids 4 gemäß $V=f_{ZP}(P)$ und $V=f_{AP}(P)$ eingerichtet. Der Beatmungsvorgang findet innerhalb eines Druckintervalls 9 statt. Durch die Steuereinrichtung 6 ist der Beatmungsvorgang so einstellbar, dass über zumindest 60 % des Druckintervalls 9 ein Verhältnis aus einem Betrag eines Maßes der Veränderung des bei einem Druck $P_0$ 11 vorliegenden ersten Volumens 12 während des Zuführens des Fluids 4, also $df_{AP}/d(P)$ $(P_0)$ (also erste Steigung 23 des ersten Kurvenabschnitts 18 bei dem Druck $P_0$ 11) und einem Betrag eines Maßes der Veränderung eines bei demselben Druck $P_0$ 11 vorliegenden zweiten Volumens 13 während des Abführens des Fluids 4, also $df_{ZP}/d(P)$ $(P_0)$ (also zweite Steigung 24 des zweiten Kurvenabschnitts 19 bei dem Druck $P_0$ 11), einen Wert von mindestens 0,5 und höchstens 2,0 aufweist.

Gleiches gilt entsprechend für jedes Volumen $V_0$ 14 (hier nicht gezeigt), wobei über zumindest 60 %

des Volumenintervalls 10 ein Verhältnis aus einem Betrag eines Maßes der Veränderung des bei einem Volumen $V_0$ 14 vorliegenden ersten Drucks 15 während des Zuführens des Fluids 4, also $df_{AV}/d(V)$ $(V_0)$ und einem Betrag eines Maßes der Veränderung eines bei demselben Volumen $V_0$ 14 vorliegenden zweiten Drucks 16 während des Abführens des Fluids 4, also $df_{ZV}/d(V)$ $(V_0)$ einen Wert von mindestens 0,5 und höchstens 2,0 aufweist.

Hier wird durch die Steuereinrichtung 6 der Beatmungsvorgang also so eingestellt, dass über zumindest einen Anteil des Druckintervalls 9 ein Verhältnis aus einem Betrag einer ersten Steigung des ersten Kurvenabschnitts 18 bei einem Druck $P_0$ 11, also $df_{AP}/d(P)$ $(P_0)$ und einem Betrag einer zweiten Steigung 24 des zweiten Kurvenabschnitts 19, also $df_{ZP}/d(P)$ $(P_0)$, bei demselben Druck $P_0$ 11, einen Wert von mindestens 0,5 und höchstens 2,0 aufweist. Der Betrag des Ergebnisses der Gleichung $[df_{AP}/d(P)$ $(P_0)]$ / $[df_{ZP}/d(P)$ $(P_0)]$ soll also mindestens 0,5 und höchstens 2,0 betragen. Dabei ist der Druck $P_0$ 11 also jeder Druck 7 innerhalb des Druckintervalls 9 bzw. innerhalb eines Anteils des Druckintervalls 9.

Hier ist der Beatmungsvorgang weiter so eingestellt, dass während des Zuführens des Fluids 4 ein bei dem Druck $P_0$ 11 vorliegendes erstes Volumen 12 und während des Abführens des Fluids 4 ein bei demselben Druck $P_0$ 11 vorliegendes zweites Volumen 13 sich höchstens um 20 % des in dem Druckintervall 9 zugeführten oder abgeführten Volumens 8 unterscheidet.

Hier ist der Beatmungsvorgang zudem so eingestellt, dass während des Zuführens des Fluids 4 ein bei dem Druck $P_0$ 11 vorliegendes erstes Volumen 12 und während des Abführens des Fluids 4 ein bei demselben Druck $P_0$ 11 vorliegendes zweites Volumen 13 sich mindestens um 1 % des in dem Druckintervall (insgesamt) zugeführten oder abgeführten Volumens 8 (hier Atemzugvolumen $V_T$ 38) unterscheidet.

Über die Visualisierungseinrichtung 17 sind nun z. B. zumindest einzelne der folgenden Parameter visuell darstellbar: ein Maß für eine Größe der Fläche 20; ein Maß für eine Veränderung der Fläche 20 über mehrere Beatmungsvorgänge hinweg; ein Maß für ein Verhältnis der Fläche 20 zu einer für einen bestimmten Patienten festgelegten kritischen Fläche 21 (also die kritische Differenz der Integrale); ein Maß für eine Veränderung des Verhältnisses der Fläche 20 zu einer für einen bestimmten Patienten festgelegten kritischen Fläche 21 (also die kritische Differenz der Integrale) über mehrere Beatmungsvorgänge hinweg. Weiter können anhand der Visualisierungseinrichtung 17 und aufgrund der Darstellung z. B. eines Beatmungsvorgangs in einem Volumen-Druck Diagramm gemäß Fig. 3 oder Fig. 4 die Steigungen 23, 24 der Kurvenabschnitte 18, 19 eingestellt bzw. verändert werden, entweder über die Steuereinrichtung 6 oder über einen Bediener der Beatmungsvorrichtung 1.

Fig. 4 zeigt auch, dass die Steuereinrichtung 6 zur Ermittlung eines Verlaufs des Druckes P 7 in dem Atemweg 5 und eines Verlaufs eines dem Atemweg 5 zugeführten und eines aus dem Atemweg 5 abgeführten Volumens V 8 des Fluids 4 für eine Compliance 25 des Patienten gemäß einem von $V=f_{CP}(P)$ und $P=f_{CV}(V)$ eingerichtet ist. Dabei entspricht hier der erste Kurvenabschnitt 18 der Compliance 25. Der Beatmungsvorgang ist durch die Steuereinrichtung 6 so einstellbar ist, dass über zumindest 60 % des Druckintervalls 9 ein Verhältnis aus jedem von $df_{AP}/d(P)$ $(P_0)$ (hier die zweite Steigung 24 des zweiten Kurvenabschnitts 19), $df_{ZP}/d(P)$ $(P_0)$ (hier die erste Steigung 23 des ersten Kurvenabschnitts 18 und einem Betrag eines Maßes der Veränderung des bei einem Druck $P_0$ 11 vorliegenden ersten Volumens 12 der Compliance 25, also $df_{CP}/d(P)$ $(P_0)$ einen Wert von mindestens 0,5 und höchstens 2,0 aufweist.

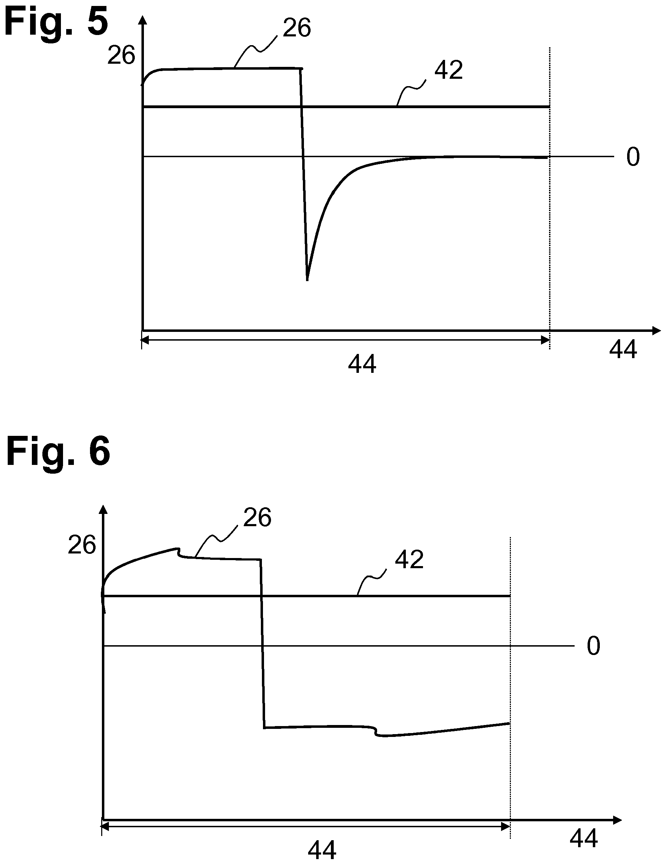

Fig. 5 zeigt ein erstes Diagramm, in dem eine Volumenflussrate 26 über der Zeit 44 dargestellt ist. Fig. 6 zeigt ein zweites Diagramm, in dem eine Volumenflussrate 26 über der Zeit 44 dargestellt ist. Fig. 5 und Fig. 6 werden im Folgenden gemeinsam beschrieben.

Die Steuereinrichtung 6 ist, wie oben ausgeführt zur Einstellung eines Verlaufs eines Druckes P 7 in dem Atemweg 5 und eines Verlaufs eines dem Atemweg 5 zugeführten und eines aus dem Atemweg 5 abgeführten Volumens V 8 des Fluids 4 gemäß $V=f_{ZP}(P)$ und $V=f_{AP}(P)$ oder gemäß $P= f_{ZV}(V)$ und $P=f_{AV}(V)$ eingerichtet, wobei der Beatmungsvorgang innerhalb eines Druckintervalls 9 und innerhalb eines Volumenintervalls 10 stattfindet. Fig. 5 zeigt die Volumenflussrate F(t) 26 des Beatmungsvorgangs nach Fig. 3. Fig. 6 zeigt die Volumenflussrate F(t) 26 des Beatmungsvorgangs nach Fig. 4. Durch die Steuereinrichtung 6 ist der Beatmungsvorgang nun so einstellbar (siehe Fig. 6), dass während des Zuführens des Fluids 4 und während des Abführens des Fluids 4 eine Volumenflussrate F(t) 26 [l/min] (hier mit unterschiedlichen Vorzeichen und gegenüber der Nulllinie also 0 l/min dargestellt) gegenüber einer, in dem Beatmungsvorgang durchschnittlichen Volumenflussrate FD 42, zumindest während 80 % einer Dauer des Beatmungsvorgangs, um höchstens 50 % variiert. Die Volumenflussrate F(t) 26 variiert erkennbar über die Zeit 44, wobei jedoch gerade eine (möglichst) konstante Volumenflussrate F(t) 26 (vom Betrag her) eingestellt werden soll. Die durchschnittliche Volumenflussrate FD 43 wird ermittelt, in dem die Summe aus zugeführten und abgeführten Fluid 4 (also immer ein positiver Wert) durch die Dauer des Beatmungsvorgangs (also die Zeit 43 zwischen dem Ursprung des Diagramms und der vertikalen Linie) dividiert wird. Die durchschnittliche Volumenflussrate FD 43 kann für die Einstellung und Steuerung der Volumenflussrate F(t) 26 auch auf Basis der vorherigen Beatmungsvorgänge oder auf Basis der voreingestellten Parameter (z. B. Frequenz und Tidalvolumen 38) ermittelt werden.

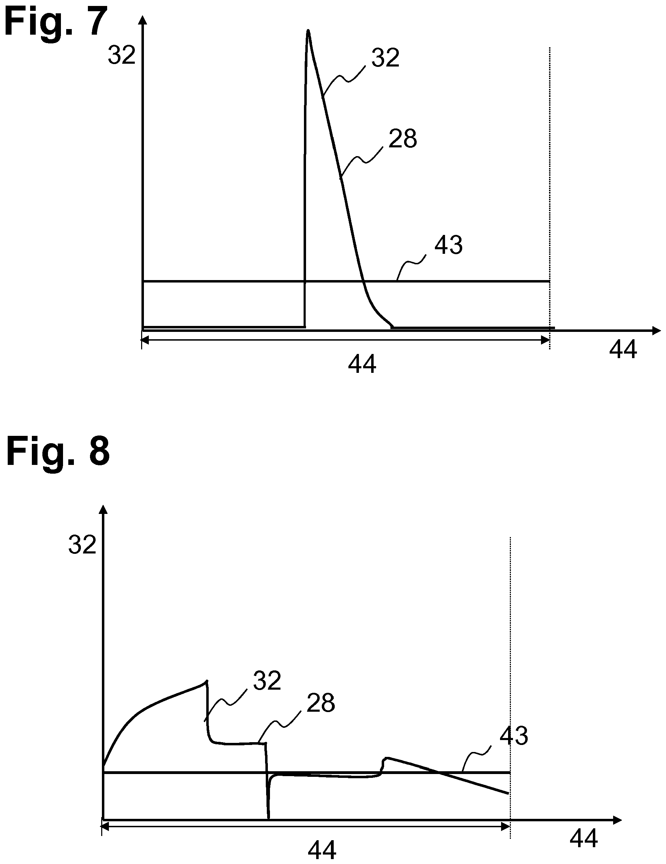

Fig. 7 zeigt ein drittes Diagramm, in dem ein Quadrat einer Geschwindigkeit über der Zeit 44 dargestellt ist. Fig. 8 zeigt ein viertes Diagramm, in dem ein Quadrat einer Geschwindigkeit über der Zeit 44 dargestellt ist. Fig. 7 und Fig. 8 werden im Folgenden gemeinsam beschrieben.

Die Steuereinrichtung 6 ist, wie oben ausgeführt zur Einstellung eines Verlaufs eines Druckes P 7 in dem Atemweg 5 und eines Verlaufs eines dem Atemweg 5 zugeführten und eines aus dem Atemweg 5 abgeführten Volumens V 8 des Fluids 4 gemäß $V=f_{ZP}(P)$ und $V=f_{AP}(P)$ oder gemäß $P=f_{ZV}(V)$ und $P=f_{AV}(V)$ eingerichtet, wobei der Beatmungsvorgang innerhalb eines Druckintervalls 9 und innerhalb eines Volumenintervalls 10 stattfindet. Fig. 7 zeigt das Quadrat einer Geschwindigkeit 32, also $(s(t))^2$, des Beatmungsvorgangs nach Fig. 3. Fig. 8 zeigt das Quadrat einer Geschwindigkeit 32, also $(s(t))^2$, des Beatmungsvorgangs nach Fig. 4. Durch die Steuereinrichtung 6 ist der Beatmungsvorgang so einstellbar, dass ein Quadrat einer Geschwindigkeit $(s(t))^2$ 32 des Verlaufs des Druckes P 7 und des Volumens V 8 während des Zuführens des Fluids 4 und während des Abführens des Fluids 4, also $(s(t))^2 = (dP/dt)^2 + (dV/dt)^2$, gegenüber einem während des Beatmungsvorgangs durchschnittlichen Quadrat einer Geschwindigkeit $sD^2$ 43 zumindest während 80 % einer Dauer des Beatmungsvorgangs, um höchstens 300 % variiert. Dabei ist erkennbar, dass in Fig. 7 ein einzelnes Maximum vorliegt, wobei in Fig. 8 das Quadrat der Geschwindigkeit 32 einen gleichmäßigeren Verlauf aufweist. Hier sind die Einheiten nicht dargestellt. Es hat sich allerdings herausgestellt, dass bei der hier beschriebenen bevorzugen Beatmungstechnik eine deutlich reduzierte Verlustleistung 28 und damit eine deutlich geringere Verlustenergie 29 während des Beatmungsvorgangs (Zeit 44) erreicht werden kann. Die Verlustleistung PW(t) 28 entspricht dem Verlauf des Quadrats einer Geschwindigkeit $(s(t))^2$ 32.

## Bezugszeichenliste

| | |
|---|---|
| 1 | Beatmungsvorrichtung |
| 2 | Fluidzuführeinheit |
| 3 | Fluidabführeinheit |
| 4 | Fluid |
| 5 | Atemweg |
| 6 | Steuereinrichtung |
| 7 | Druck P |
| 8 | Volumen V |
| 9 | Druckintervall |
| 10 | Volumenintervall |
| 11 | Druck $P_0$ |
| 12 | erstes Volumen |
| 13 | zweites Volumen |
| 14 | Volumen $V_0$ |
| 15 | erster Druck |
| 16 | zweiter Druck |
| 17 | Visualisierungseinrichtung |
| 18 | erster Kurvenabschnitt |
| 19 | zweiter Kurvenabschnitt |
| 20 | Fläche |
| 21 | kritische Fläche |
| 22 | Eingriffsgrenze |
| 23 | erste Steigung |
| 24 | zweite Steigung |
| 25 | Compliance C |
| 26 | Volumenflussrate F(t) |
| 27 | Atemwegswiderstand |
| 28 | Verlustleistung PW(t) |
| 29 | Verlustenergie E |
| 30 | kritische Verlustleistung |
| 31 | kritische Verlustenergie |
| 32 | Geschwindigkeit zum Quadrat $(s(t))^2$ |
| 33 | kritischen Geschwindigkeit zum Quadrat |
| 34 | Größe |
| 35 | Compliance-Kurve |
| 36 | Druck P1 |
| 37 | Druck P2 |
| 38 | Atemzugvolumen $V_T$ |
| 39 | Drucksensor |
| 40 | Katheter |
| 41 | Querschnitt |
| 42 | durchschnittliche Volumenflussrate |
| 43 | durchschnittliches Quadrat einer Geschwindigkeit |
| 44 | Zeit t |

## Patentansprüche

1. Beatmungsvorrichtung (1) zur Beatmung eines Patienten, zumindest umfassend eine Fluidzuführeinheit (2) und eine Fluidabführeinheit (3), die zum Zuführen (ZV, ZP) eines Fluids (4) in zumindest einen Atemweg (5), also in einen Lungenteil oder in die Lunge, eines Patienten sowie zum Abführen (AV, AP) des Fluids (4) aus diesem Atemweg (5) geeignet ist; sowie eine Steuereinrichtung (6), die, zumindest während eines Beatmungsvorgangs des zumindest einen Atemwegs (5), also dem zumindest einmaligen Zuführen des Fluids (4) in den zumindest einen Atemweg (5) und dem zumindest einmaligen Abführen des Fluids (4) aus dem zumindest einen Atemweg (5) durch Betreiben der Beatmungsvorrichtung (1), zur Einstellung eines Verlaufs eines Druckes P (7) in dem Atemweg (5) und eines Verlaufs eines dem Atemweg (5) zugeführten und eines aus dem Atemweg (5) abgeführten Volumens V (8) des Fluids (4) gemäß $V=f_{ZP}(P)$ und $V=f_{AP}(P)$ oder gemäß $P=f_{ZV}(V)$ und $P=f_{AV}(V)$ eingerichtet ist, wobei der Beatmungsvorgang innerhalb eines Druckintervalls (9) und innerhalb eines Volumenintervalls (10) stattfindet; **dadurch gekennzeichnet, dass** durch die Steuereinrichtung (6) der Beatmungsvorgang so eingestellt ist, dass

   a) über zumindest 60 % des Druckintervalls (9) ein Verhältnis aus

      - einem Betrag eines Maßes der Veränderung des bei einem Druck $P_0$ (11) vorliegen-

den ersten Volumens (12) während des Zuführens des Fluids (4), also $df_{AP}/d(P)$ ($P_0$) und

- einem Betrag eines Maßes der Veränderung eines bei demselben Druck $P_0$ (11) vorliegenden zweiten Volumens (13) während des Abführens des Fluids (4), also $df_{ZP}/d(P)$ ($P_0$)

oder

b) über zumindest 60 % des Volumenintervalls (10) ein Verhältnis aus

- einem Betrag eines Maßes der Veränderung des bei einem Volumen $V_0$ (14) vorliegenden ersten Drucks (15) während des Zuführens des Fluids (4), also $df_{AV}/d(V)$ ($V_0$) und
- einem Betrag eines Maßes der Veränderung eines bei demselben Volumen $V_0$ (14) vorliegenden zweiten Drucks (16) während des Abführens des Fluids (4), also $df_{ZV}/d(V)$ ($V_0$)

einen Wert von mindestens 0,5 und höchstens 2,0 aufweist.

2. Beatmungsvorrichtung (1) nach Patentanspruch 1, wobei die Steuereinrichtung zur Ermittlung eines Verlaufs des Druckes P (7) in dem Atemweg (5) und eines Verlaufs eines dem Atemweg (5) zugeführten und eines aus dem Atemweg (5) abgeführten Volumens V (8) des Fluids (4) für eine Compliance (25) des Patienten gemäß einem von $V=f_{CP}(P)$ und $P=f_{CV}(V)$ eingerichtet ist; wobei der Beatmungsvorgang so einstellbar ist, dass

a) über zumindest 60 % des Druckintervalls (9) ein Verhältnis aus

- jedem von $df_{AP}/d(P)$ ($P_0$), $df_{ZP}/d(P)$ ($P_0$) und
- einem Betrag eines Maßes der Veränderung des bei einem Druck $P_0$ (11) vorliegenden ersten Volumens (12) der Compliance (25), also $df_{CP}/d(P)$ ($P_0$), oder

b) über zumindest 60 % des Volumenintervalls (10) ein Verhältnis aus

- jedem von $df_{AV}/d(V)$ ($V_0$), $df_{ZV}/d(V)$ ($V_0$) und
- und einem Betrag eines Maßes der Veränderung des bei einem Volumen $V_0$ (14) vorliegenden ersten Drucks (15) der Compliance (25), also $df_{CV}/d(V)$ ($V_0$)

einen Wert von mindestens 0,5 und höchstens 2,0 aufweist.

3. Beatmungsvorrichtung (1) nach einem der vorhergehenden Patentansprüche, wobei der Beatmungsvorgang so einstellbar ist, dass über zumindest 60 % des Druckintervalls (9) oder 60 % des Volumenintervalls (10) das Verhältnis einen Wert von mindestens 0,67 und höchstens 1,5 aufweist.

4. Beatmungsvorrichtung (1) nach einem der vorhergehenden Patentansprüche, wobei der Beatmungsvorgang so einstellbar ist, dass über zumindest 60 % des Druckintervalls (9) oder 60 % des Volumenintervalls (10) das Verhältnis größer oder kleiner als 1,0 ist.

5. Beatmungsvorrichtung (1) nach einem der vorhergehenden Patentansprüche, wobei der Beatmungsvorgang so einstellbar ist, dass

a) während des Zuführens des Fluids (4) ein bei dem Druck $P_0$ (11) vorliegendes erstes Volumen (12) und während des Abführens des Fluids (4) ein bei demselben Druck $P_0$ (11) vorliegendes zweites Volumen (13) sich höchstens um 30 % des in dem Druckintervall (9) vorliegenden Volumenintervalls (10) unterscheidet oder
b) während des Zuführens des Fluids (4) ein bei dem Volumen $V_0$ (14) vorliegender erster Druck (15) und während des Abführens des Fluids (4) ein bei demselben Volumen $V_0$ (14) vorliegender zweiter Druck (16) sich höchstens um 30 % des in dem Volumenintervall (10) vorliegenden Druckintervalls (9) unterscheidet.

6. Beatmungsvorrichtung (1) nach einem der vorhergehenden Patentansprüche, wobei

a) der Beatmungsvorgang über zumindest 60 % des Druckintervalls (9) so einstellbar ist, dass während des Zuführens des Fluids (4) ein bei dem Druck $P_0$ (11) vorliegendes erstes Volumen (12) und während des Abführens des Fluids (4) ein bei demselben Druck $P_0$ (11) vorliegendes zweites Volumen (13) sich mindestens um 1 % des in dem Druckintervall (9) vorliegenden Volumenintervalls (10) unterscheidet oder
b) der Beatmungsvorgang über zumindest 60 % des Volumenintervalls (10) so einstellbar ist, dass während des Zuführens des Fluids (4) ein bei dem Volumen $V_0$ (14) vorliegender erster Druck (15) und während des Abführens des Fluids (4) ein bei demselben Volumen $V_0$ (14) vorliegender zweiter Druck (16) sich mindestens um 1 % des in dem Volumenintervall (10) vorliegenden Druckintervalls (9) unterscheidet.

7. Beatmungsvorrichtung (1) nach einem der vorhergehenden Patentansprüche, wobei die Steuereinrichtung (6)

a) zur Bestimmung von Integralen von $f_{ZP}(P)$ und $f_{AP}(P)$ in dem Druckintervall (9) sowie zur Bestimmung einer Differenz von $\int f_{ZP}(P)\,dP$ und $\int f_{AP}(P)\,dP$ in dem Druckintervall (9) oder
b) zur Bestimmung von Integralen von $f_{ZV}(V)$ und $f_{AV}(V)$ in dem Volumenintervall (10) sowie zur Bestimmung einer Differenz von $\int f_{ZV}(V)\,dV$ und $\int f_{AV}(V)\,dV$ in dem Volumenintervall (10) ausgebildet ist.

8. Beatmungsvorrichtung (1) nach Patentanspruch 7, wobei die Steuereinrichtung (6) zur Durchführung von mehreren Beatmungsvorgängen ausgebildet ist, bei denen die Differenz von

a) $\int f_{ZP}(P)\,dP$ und $\int f_{AP}(P)\,dP$ in dem Druckintervall (9) oder
b) $\int f_{ZV}(V)\,dV$ und $\int f_{AV}(V)\,dV$ in dem Volumenintervall (10) geregelt ist, wobei ein Verhältnis der Differenz zu einer für einen bestimmten Patienten festgelegten kritischen Differenz eingestellt ist.

9. Beatmungsvorrichtung (1) nach einem der vorhergehenden Patentansprüche, mit einer Visualisierungseinrichtung (17), wobei die Steuereinrichtung (6) zur Ermittlung eines Verlaufs zumindest einer Volumen-Druck Kurve in einem Volumen-Druck Diagramm ausgebildet ist; wobei die Kurve einen ersten Kurvenabschnitt (18), $V=f_{ZP}(P)$ oder $P=f_{ZV}(V)$, und einen zweiten Kurvenabschnitt (19), $V=f_{AP}(P)$ oder $P=f_{AV}(V)$, aufweist, wobei der erste Kurvenabschnitt (18) den Verlauf des zugeführten Volumens V (8) und des Druckes P (7) während des Zuführen des Fluids (4) in den zumindest einen Atemweg und der zweite Kurvenabschnitt (19) den Verlauf des abgeführten Volumens V (8) und des Druckes P (7) während des Abführens des Fluids (4) aus dem zumindest einen Atemweg (5) darstellt; wobei der Beatmungsvorgang innerhalb eines Druckintervalls (9) und innerhalb eines Volumenintervalls (10) stattfindet; wobei die Steuereinrichtung (6) zur Bestimmung einer Fläche (20) ausgebildet ist, wobei diese Fläche (20) in dem Volumen-Druck Diagramm von dem ersten Kurvenabschnitt (18) und dem zweiten Kurvenabschnitt (19) des einen Beatmungsvorgangs umschlossen ist; wobei über die Visualisierungseinrichtung (17) zumindest einer der folgenden Parameter:

a) ein Maß für eine Größe der Fläche (20); oder
b) ein Maß für eine Veränderung der Fläche (20) über mehrere Beatmungsvorgänge hinweg; oder
c) ein Maß für ein Verhältnis der Fläche (20) zu einer für einen bestimmten Patienten festgelegten kritischen Fläche (21); oder
d) ein Maß für eine Veränderung des Verhältnisses der Fläche (20) zu einer für einen bestimmten Patienten festgelegten kritischen Fläche (21) über mehrere Beatmungsvorgänge hinweg visuell erkennbar dargestellt ist.

10. Beatmungsvorrichtung (1) nach Patentanspruch 9, wobei zumindest einer der Parameter a. bis d. in Relation zu zumindest einer Eingriffsgrenze (22) darstellbar ist.

11. Beatmungsvorrichtung (1) nach einem der vorhergehenden Patentansprüche, wobei durch die Steuereinrichtung (6) der Beatmungsvorgang so eingestellt ist, dass während des Zuführen des Fluids (4) und während des Abführens des Fluids (4) eine Volumenflussrate F(t) (26) gegenüber einer, in dem Beatmungsvorgang durchschnittlichen Volumenflussrate FD (42), zumindest während 80 % einer Dauer des Beatmungsvorgangs, um höchstens 50 % variiert.

12. Beatmungsvorrichtung (1) nach einem der Patentansprüche 9 bis 11, wobei die Steuereinrichtung (6) zumindest während eines Beatmungsvorgangs des zumindest einen Atemwegs (5), zur Ermittlung und Einstellung einer Volumenflussrate F(t) (26) des Fluids (4) ausgebildet ist; wobei der Beatmungsvorgang innerhalb eines Druckintervalls (9) und innerhalb eines Volumenintervalls (10) stattfindet; wobei die Steuereinrichtung (6) unter Annahme eines Atemwegswiderstands R (27) des Atemwegs (5) des Patienten zur Bestimmung einer Verlustleistung PW(t) (28) [Watt] des Atemwegs (5) gemäß $PW(t) = R_1 \cdot (F(t))^3 + R_2 \cdot (F(t))^2$, mit $R_1 = R$ in Pascal/$(m^3/s)^2$ und $R_2 = R$ in Pascal/$(m^3/s)$ ausgebildet ist; wobei über die Visualisierungseinrichtung (17) zumindest einer der folgenden Parameter:

a) die Verlustleistung PW(t) (28); oder
b) eine Verlustenergie E (29), nämlich das Integral von PW(t)dt, also $\int PW(t)\,dt$ in einem Zeitintervall; oder
c) ein Maß für ein Verhältnis der Verlustleistung PW(t) (28) zu einer für einen bestimmten Patienten festgelegten kritischen Verlustleistung (30); oder
d) ein Maß für ein Verhältnis der Verlustenergie E (29) zu einer für einen bestimmten Patienten festgelegten kritischen Verlustenergie (31) visuell erkennbar dargestellt ist.

13. Beatmungsvorrichtung (1) nach einem der vorhergehenden Patentansprüche, wobei durch die Steuereinrichtung (6) der Beatmungsvorgang so eingestellt ist, dass ein Quadrat einer Geschwindigkeit $(s(t))^2$ (32) des Verlaufs des Druckes P (7) $[cmH_2O]$

und des Volumens V (8) [ml] während des Zuführens des Fluids (4) und während des Abführen des Fluids (4), also $(s(t))^2 = (dP/dt)^2 + (dV/dt)^2$, gegenüber einem während des Beatmungsvorgangs durchschnittlichen Quadrat einer Geschwindigkeit $sD^2$ (43) zumindest während 80 % einer Dauer des Beatmungsvorgangs, um höchstens 300 % variiert.

14. Beatmungsvorrichtung (1) nach einem der Patentansprüche 9 bis 13, mit einer Visualisierungseinrichtung (13), wobei die Steuereinrichtung (6) zumindest während eines Beatmungsvorgangs des zumindest einen Atemwegs (5) zur Ermittlung eines Verlaufs zumindest einer Volumen-Druck Kurve in einem Volumen-Druck Diagramm ausgebildet ist; wobei die Kurve einen ersten Kurvenabschnitt (18), $V = f_{ZP}(P)$ oder $P = f_{ZV}(V)$, und einen zweiten Kurvenabschnitt (19), $V = f_{AP}(P)$ oder $P = f_{AV}(V)$, aufweist, wobei der erste Kurvenabschnitt (18) den Verlauf des zugeführten Volumens V (8) und des Druckes P (7) während des Zuführen des Fluids (4) in den zumindest einen Atemweg (5) und der zweite Kurvenabschnitt (19) den Verlauf des abgeführten Volumens V (8) und des Druckes P (7) während des Abführens des Fluids (4) aus dem zumindest einen Atemweg (5) darstellt; wobei der Beatmungsvorgang innerhalb eines Druckintervalls (9) und innerhalb eines Volumenintervalls (10) stattfindet; wobei die Steuereinrichtung (6) zur Bestimmung eines Quadrats einer Geschwindigkeit $(s(t))^2$ (32) des Verlaufs des Druckes P (7) [cmH$_2$O] und des Volumens V (8) [ml] während des Zuführens des Fluids (4) und während des Abführen des Fluids (4), also $(s(t))^2 = (dP/dt)^2 + (dV/dt)^2$ ausgebildet ist, wobei über die Visualisierungseinrichtung (17) zumindest einer der folgenden Parameter:

　　a) das Quadrat der Geschwindigkeit s(t), also $(s(t))^2$ oder
　　b) das Integral von $(s(t))^2 dt$, also $\int (s(t))^2 dt$ in einem Zeitintervall; oder
　　c) ein Maß für ein Verhältnis des Quadrats der Geschwindigkeit $(s(t))^2$ (32) zu einer für einen bestimmten Patienten festgelegten kritischen Geschwindigkeit zum Quadrat (33); oder
　　d) ein Maß für ein Verhältnis des Integrals von $(s(t))^2 dt$ zu einem für einen bestimmten Patienten festgelegten kritischen Wert dieser Größe (34)

visuell erkennbar dargestellt ist.

## Claims

1. Ventilation device (1) for ventilating a patient, comprising at least a fluid supply unit (2) and a fluid discharge unit (3) that are suitable for respectively supplying (ZV, ZP) a fluid (4) into at least one airway (5), i.e., into a lung part or into the lung, of a patient and for discharging (AV, AP) the fluid (4) from this airway (5); and further comprising a control device (6) which, at least during a ventilation process for the at least one airway (5), i.e., during the at least one-time supply of the fluid (4) into the at least one airway (5) and the at least one-time discharge of the fluid (4) from the at least one airway (5) by operating the ventilation device (1), is configured for setting a profile of a pressure P (7) in the airway (5), and a profile of a volume V (8) of the fluid (4) supplied to the airway (5) and discharged from the airway (5) according to $V = f_{ZP}(P)$ and $V = f_{AP}(P)$, or according to $P = f_{ZV}(V)$ and $P = f_{AV}(V)$, wherein the ventilation process takes place within a pressure interval (9) and within a volume interval (10); **characterized in that** the ventilation process is set by the control device (6) in such a way that

　　a) over at least 60% of the pressure interval (9), a ratio

　　　　- of an absolute value of a measure of the change in the first volume (12) that is present at a pressure $P_0$ (11) while supplying the fluid (4), i.e., $df_{AP}/d(P)$ (P0) and
　　　　- an absolute value of a measure of the change in a second volume (13) that is present at the same pressure $P_0$ (11) while discharging the fluid (4), i.e., $df_{ZP}/d(P)$ (P0) or

　　b) over at least 60% of the volume interval (10), a ratio of

　　　　- an absolute value of a measure of the change in the first pressure (15) that is present at a volume $V_0$ (14) while supplying the fluid (4), i.e., $df_{AV}/d(V)$ (V0) and
　　　　- an absolute value of a measure of the change in a second pressure (16) that is present at the same volume V0 (14) while discharging the fluid (4), i.e., $df_{ZV}/d(V)$ (V0),

　　has a value of at least 0.5 and at most 2.0.

2. Ventilation device (1) according to Claim 1, wherein the control device is configured for determining a profile of the pressure P (7) in the airway (5) and of a profile of a volume V (8) of the fluid (4) that is supplied to the airway (5) and discharged from the airway (5) for compliance (25) of the patient according to one of $V = f_{CP}(P)$ or $P = f_{CV}(V)$, wherein the ventilation process is settable in such a way that

　　a) over at least 60% of the pressure interval (9), a ratio of

- each of $df_{AP}/d(P)$ (P0), $df_{ZP}/d(P)$ (P0) and
- an absolute value of a measure of the change in the first volume (12) of the compliance (25) that is present at a pressure $P_0$ (11), i.e., $df_{CP}/d(P)$ (P0), or

b) over at least 60% of the volume interval (10), a ratio of

- each of $df_{AV}/d(V)$ (V0), $df_{ZV}/d(V)$ (V0) and
- an absolute value of a measure of the change in the first pressure (15) of the compliance (25) that is present at a volume $V_0$ (14), i.e., $df_{CV}/d(V)$ (V0),

has a value of at least 0.5 and at most 2.0.

3. Ventilation device (1) according to one of the preceding claims, wherein the ventilation process is settable in such a way that over at least 60% of the pressure interval (9) or 60% of the volume interval (10), the ratio has a value of at least 0.67 and at most 1.5.

4. Ventilation device (1) according to one of the preceding claims, wherein the ventilation process is settable in such a way that over at least 60% of the pressure interval (9) or 60% of the volume interval (10), the ratio is greater or lesser than 1.0.

5. Ventilation device (1) according to one of the preceding claims, wherein the ventilation process is settable in such a way that

a) while supplying the fluid (4), a first volume (12) that is present at the pressure $P_0$ (11), and while discharging the fluid (4), a second volume (13) that is present at the same pressure $P_0$ (11), differs at most by 30% from the volume interval (10) that is present in the pressure interval (9) or
b) while supplying the fluid (4), a first pressure (15) that is present at the volume $V_0$ (14), and while discharging the fluid (4), a second pressure (16) that is present at the same volume $V_0$ (14), differs at most by 30% from the pressure interval (9) that is present in the volume interval (10).

6. Ventilation device (1) according to one of the preceding claims, wherein

a) the ventilation process is settable over at least 60% of the pressure interval (9) in such a way that while supplying the fluid (4), a first volume (12) that is present at the pressure $P_0$ (11), and while discharging the fluid (4), a second volume (13) that is present at the same pressure $P_0$ (11), differs at least by 1 % from the volume interval (10) that is present in the pressure interval (9) or
b) the ventilation process is settable over at least 60% of the volume interval (10) in such a way that while supplying the fluid (4), a first pressure (15) that is present at the volume $V_0$ (14), and while discharging the fluid (4), a second pressure (16) that is present at the same volume $V_0$ (14), differs at least by 1 % from the pressure interval (9) that is present in the volume interval (10).

7. Ventilation device (1) according to one of the preceding claims, wherein the control device (6) is configured

a) for determining integrals of $f_{ZP}(P)$ and $f_{AP}(P)$ in the pressure interval (9) and for determining a difference between $\int f_{ZP}(P)\, dP$ and $\int f_{AP}(P)\, dP$ in the pressure interval (9) or
b) for determining integrals of $f_{ZV}(V)$ and $f_{AV}(V)$ in the volume interval (10) and for determining a difference between $\int f_{ZV}(V)\, dV$ and $\int f_{AV}(V)\, dV$ in the volume interval (10).

8. Ventilation device (1) according to Claim 7, wherein the control device (6) is configured for carrying out multiple ventilation processes, in which the difference between

a) $\int f_{ZP}(P)\, dP$ and $\int f_{AP}(P)\, dP$ in the pressure interval (9) or
b) $\int f_{ZV}(V)\, dV$ and $\int f_{AV}(V)\, dV$ in the volume interval (10) is controlled, wherein a ratio of the difference to a critical difference that is established for a given patient is set.

9. Ventilation device (1) according to one of the preceding claims having a visualization apparatus (17), the control device (6), is configured for determining a profile of at least one volume-pressure curve in a volume-pressure diagram; wherein the curve has a first curve section (18), $V = f_{ZP}(P)$ or $P = f_{ZV}(V)$, and a second curve section (19), $V = f_{AP}(P)$ or $P = f_{AV}(V)$, wherein the first curve section (18) represents the profile of the supplied volume V (8) and of the pressure P (7) while supplying the fluid (4) into the at least one airway, and the second curve section (19) represents the profile of the discharged volume V (8) and of the pressure P (7) while discharging the fluid (4) from the at least one airway (5); wherein the ventilation process takes place within a pressure interval (9) and within a volume interval (10); wherein the control device (6) is configured for determining an area (20), this area (20) in the volume-pressure diagram being enclosed by the first curve section (18) and the second curve section (19) of the one ventilation process; wherein at least one of the following parameters is visually discernibly displayed via the visualization apparatus (17):

> a) a measure for a size of the area (20); or
> b) a measure for a change in the area (20) over multiple ventilation processes; or
> c) a measure for a ratio of the area (20) to a critical area (21) that is established for a given patient; or
> d) a measure for a change in the ratio of the area (20) to a critical area (21) that is established for a given patient over multiple ventilation processes.

**10.** Ventilation device (1) according to Claim 9, wherein at least one of the parameters a. through d. is displayable in relation to at least one intervention limit (22).

**11.** Ventilation device (1) according to one of the preceding claims, wherein the ventilation process is set by the control device (6) in such a way that while supplying the fluid (4) and while discharging the fluid (4), a volumetric flow rate F(t) (26) varies at most by 50% with respect to an average volumetric flow rate FD (42) in the ventilation process, at least for 80% of a duration of the ventilation process.

**12.** Ventilation device (1) according to one of the preceding claims 9 to 11, wherein the control device (6), at least during a ventilation process of the at least one airway (5) is configured for determining and setting a volumetric flowrate F(t) (26) of the fluid (4); wherein the ventilation process takes place within a pressure interval (9) and within a volume interval (10); wherein the control device (6), assuming an airway resistance R (27) of the airway of the patient (5), is configured for determining a power loss PW(t) (28) of the airway according to $PW(t) = R1*(F(t))^3 + R_2*(F(t))^2$, where $R_1 = R$ in $Pascal/(m^3/s)^2$ and $R_2 = R$ in $Pascal/(m^3/s)$; wherein at least one of the following parameters is visually discernibly displayed via the visualization apparatus (17):

> a) the power loss PW(t) (28); or
> b) an energy loss E (29), namely, the integral of PW(t)dt, i.e., $\int PW(t)dt$ in a time interval; or
> c) a measure for a ratio of the power loss PW(t) (28) to a critical power loss (30) that is established for a given patient; or
> d) a measure for a ratio of the energy loss E (29) to a critical energy loss (31) that is established for a given patient.

**13.** Ventilation device (1) according to one of the preceding claims, wherein the ventilation process is set by the control device (6) in such a way that while supplying the fluid (4) and while discharging the fluid (4), the square of a speed $(s(t))^2$ (32) of the profile of the pressure P (7) [cm H2O] and of the volume V (8) [mL], i.e., $(s(t))^2 = (dP/dt)^2 + (dV/dt)^2$, varies at most by 300% with respect to an average square of a speed $sD^2$ (43) during the ventilation process, at least for 80% of a duration of the ventilation process.

**14.** Ventilation device (1) according to one of the preceding claims 9 to 13, wherein the control device (6), at least during a ventilation process of the at least one airway (5), is configured for determining a profile of at least one volume-pressure curve in a volume-pressure diagram; wherein the curve has a first curve section (18), $V = f_{ZP}(P)$ or $P = f_{ZV}(V)$, and a second curve section (19), $V = f_{AP}(P)$ or $P = f_{AV}(V)$, wherein the first curve section (18) represents the profile of the supplied volume V (8) and of the pressure P (7) while supplying the fluid (4) into the at least one airway (5), and the second curve section (19) represents the profile of the discharged volume V (8) and of the pressure P (7) while discharging the fluid (4) from the at least one airway (5); wherein the ventilation process takes place within a pressure interval (9) and within a volume interval (10); wherein the control device (6) is configured for determining the square of a speed $(s(t))^2$ (32) of the profile of the pressure P (7) [cm H2O] and of the volume V (8) [mL] while supplying the fluid (4) and while discharging the fluid (4), i.e., $(s(t))^2 = (dP/dt)^2 + (dV/dt)^2$, wherein at least one of the following parameters is visually discernibly displayed via the visualization apparatus (17):

> a) the square of the speed s(t), i.e., $(s(t))^2$ or
> b) the integral of $(s(t))^2dt$, i.e., $\int (s(t))^2dt$ in a time interval; or
> c) a measure for a ratio of the square of the speed $(s(t))^2$ (32) to a critical speed squared (33) that is established for a given patient; or
> d) a measure for a ratio of the integral of $(s(t))^2dt$ to a critical value of this variable (34) that is established for a given patient.

## Revendications

**1.** Dispositif de ventilation (1) pour la ventilation d'un patient, comprenant au moins une unité d'amenée de fluide (2) et une unité d'évacuation de fluide (3), qui est appropriée pour l'amenée (ZV, ZP) d'un fluide (4) dans au moins une voie respiratoire (5), c'est-à-dire dans une partie de poumon ou dans les poumons, d'un patient ainsi que pour l'évacuation (AV, AP) du fluide (4) de cette voie respiratoire (5) ; ainsi qu'un appareil de commande (6) qui, au moins pendant une opération de ventilation de l'au moins une voie respiratoire (5), c'est-à-dire l'amenée à au moins une reprise du fluide (4) dans l'au moins une voie respiratoire (5) et l'évacuation à au moins une reprise du fluide (4) de l'au moins une voie respiratoire (5) par exploitation du dispositif de ventilation (1), pour ajuster une évolution d'une pression P (7) dans la voie respiratoire (5) et une évolution d'un volume V (8) du fluide (4) amené à la voie respiratoire (5) et évacué de la voie respiratoire (5), est conçu selon $V = f_{ZP}(P)$ et $V = f_{AP}(P)$ ou selon $P = f_{ZV}(V)$ et $P = f_{AV}(V)$, l'opération de ventilation ayant lieu dans un intervalle de pression (9) et dans un intervalle de volume (10) ; **caractérisé en ce que** l'opération de ventilation est ajustée par l'appareil de commande (6) de telle sorte que

> a) sur au moins 60 % de l'intervalle de pression (9), un rapport entre
>
> > - un montant d'une mesure de la variation du premier volume (12) présent à une pression $P_0$ (11) pendant l'amenée du fluide (4), c'est-à-dire $df_{AP}/d(P)(P_0)$ et
> > - un montant d'une mesure de la variation d'un deuxième volume (13) présent à la même pression $P_0$ (11) pendant l'évacuation du fluide (4), c'est-à-dire $df_{ZP}/d(P)(P_0)$,
>
> ou
>
> b) sur au moins 60 % de l'intervalle de volume (10), un rapport entre
>
> > - un montant d'une mesure de la variation de la première pression (15) présente à un volume $V_0$ (14) pendant l'amenée du fluide (4), c'est-à-dire $df_{AV}/d(V)(V_0)$ et
> > - un montant d'une mesure de la variation d'une deuxième pression (16) présente au même volume $V_0$ (14) pendant l'évacuation du fluide (4), c'est-à-dire $df_{ZV}/d(V)(V_0)$,
>
> présente une valeur d'au moins 0,5 et d'au plus 2,0.

**2.** Dispositif de ventilation (1) selon la revendication 1, l'appareil de commande étant conçu pour déterminer une évolution de la pression P (7) dans la voie respiratoire (5) et une évolution d'un volume V (8) du fluide (4) amené à la voie respiratoire (5) et évacué de la voie respiratoire (5) pour une compliance (25) du patient selon $V = f_{CP}(P)$ ou $P = f_{CV}(V)$ ; l'opération de ventilation étant ajustable de telle sorte que

> a) sur au moins 60 % de l'intervalle de pression (9), un rapport entre
>
> > - chacun de $df_{AP}/d(P)(P_0)$, $df_{ZP}/d(P)(P_0)$ et
> > - un montant d'une mesure de la variation du premier volume (12) de la compliance (25) présent à une pression $P_0$ (11), c'est-à-dire $df_{CP}/d(P)(P_0)$, ou

> b) sur au moins 60 % de l'intervalle de volume (10), un rapport entre
>
> > - chacun de $df_{AV}/d(V)(V_0)$, $df_{ZV}/d(V)(V_0)$ et
> > - un montant d'une mesure de la variation de la première pression (15) de la compliance (25) présente à un volume $V_0$ (14), c'est-à-dire $df_{CV}/d(V)(V_0)$,
>
> présente une valeur d'au moins 0,5 et d'au plus 2,0.

**3.** Dispositif de ventilation (1) selon l'une quelconque des revendications précédentes, l'opération de ventilation étant ajustable de telle sorte que sur au moins 60 % de l'intervalle de pression (9) ou 60 % de l'intervalle de volume (10), le rapport présente une valeur d'au moins 0,67 et d'au plus 1,5.

**4.** Dispositif de ventilation (1) selon l'une quelconque des revendications précédentes, l'opération de ventilation étant ajustable de telle sorte que sur au moins 60 % de l'intervalle de pression (9) ou 60 % de l'intervalle de volume (10), le rapport est supérieur ou inférieur à 1,0.

**5.** Dispositif de ventilation (1) selon l'une quelconque des revendications précédentes, l'opération de ventilation étant ajustable de telle sorte que a) pendant l'amenée du fluide (4), un premier volume (12) présent à la pression $P_0$ (11) et pendant l'évacuation du fluide (4), un deuxième volume (13) présent à la même pression $P_0$ (11) diffèrent au plus de 30 % de l'intervalle de volume (10) présent dans l'intervalle de pression (9), ou b) pendant l'amenée du fluide (4), une première pression (15) présente au volume $V_0$ (14) et, pendant l'évacuation du fluide (4), une deuxième pression (16) présente au même volume $V_0$ (14) diffèrent au plus de 30 % de l'intervalle de pression (9) présent dans l'intervalle de volume (10).

**6.** Dispositif de ventilation (1) selon l'une quelconque des revendications précédentes,

> a) l'opération de ventilation étant ajustable sur au moins 60 % de l'intervalle de pression (9) de telle sorte que, pendant l'amenée du fluide (4), un premier volume (12) présent à la pression $P_0$ (11) et, pendant l'évacuation du fluide (4), un deuxième volume (13) présent à la même pression $P_0$ (11) diffèrent d'au moins 1 % de l'intervalle de volume (10) présent dans l'intervalle de pression (9) ou
>
> b) l'opération de ventilation étant ajustable sur au moins 60 % de l'intervalle de volume (10) de telle sorte que, pendant l'amenée du fluide (4), une première pression (15) présente au volume $V_0$ (14) et, pendant l'évacuation du fluide (4), une deuxième pression (16) présente au même volume $V_0$ (14) diffèrent d'au moins 1 % de l'intervalle de pression (9) présent dans l'intervalle de volume (10).

**7.** Dispositif de ventilation (1) selon l'une quelconque des revendications précédentes, l'appareil de commande (6) étant configuré

a) pour déterminer des intégrales de $f_{ZP}(P)$ et $f_{AP}(P)$ dans l'intervalle de pression (9) ainsi que pour déterminer une différence entre $\int f_{ZP}(P)dP$ et $\int f_{AP}(P)dP$ dans l'intervalle de pression (9) ou

b) pour déterminer des intégrales de $f_{ZV}(V)$ et $f_{AV}(V)$ dans l'intervalle de volume (10) ainsi que pour déterminer une différence entre $\int f_{ZV}(V)dV$ et $\int f_{AV}(V)dV$ dans l'intervalle de volume (10).

**8.** Dispositif de ventilation (1) selon la revendication 7, l'appareil de commande (6) étant configuré pour effectuer plusieurs opérations de ventilation dans lesquelles la différence entre

a) $\int f_{ZP}(P)dP$ et $\int f_{AP}(P)dP$ dans l'intervalle de pression (9) ou

b) $\int f_{ZV}(V)dV$ et $\int f_{AV}(V)dV$ dans l'intervalle de volume (10) est régulée, un rapport entre la différence et une différence critique définie pour un patient déterminé étant ajustée.

**9.** Dispositif de ventilation (1) selon l'une quelconque des revendications précédentes, comprenant un appareil de visualisation (17), l'appareil de commande (6) étant configuré pour déterminer une évolution d'au moins une courbe volume-pression dans un diagramme volume-pression ; la courbe présentant une première section de courbe (18), $V = f_{ZP}(P)$ ou $P = f_{ZV}(V)$, et une deuxième section de courbe (19), $V = f_{AP}(P)$ ou $P = f_{AV}(V)$, la première section de courbe (18) représentant l'évolution du volume amené $V$ (8) et de la pression $P$ (7) pendant l'amenée du fluide (4) dans l'au moins une voie respiratoire et la deuxième section de courbe (19) représentant l'évolution du volume évacué $V$ (8) et de la pression $P$ (7) pendant l'évacuation du fluide (4) de l'au moins une voie respiratoire (5) ; l'opération de ventilation ayant lieu dans un intervalle de pression (9) et dans un intervalle de volume (10) ; l'appareil de commande (6) étant configuré pour déterminer une surface (20), cette surface (20) étant entourée dans le diagramme volume-pression par la première section de courbe (18) et la deuxième section de courbe (19) de l'opération de ventilation ; au moins l'un des paramètres suivants étant représenté de manière visuellement reconnaissable par l'appareil de visualisation (17) :

a) une mesure d'une dimension de la surface (20) ; ou

b) une mesure d'une variation de la surface (20) sur plusieurs opérations de ventilation ; ou

c) une mesure d'un rapport entre la surface (20) et une surface critique (21) définie pour un patient déterminé ; ou

d) une mesure d'une variation du rapport entre la surface (20) et une surface critique (21) définie pour un patient déterminé sur plusieurs opérations de ventilation.

**10.** Dispositif de ventilation (1) selon la revendication 9, au moins un des paramètres a. à d. pouvant être représenté par rapport à au moins une limite d'intervention (22).

**11.** Dispositif de ventilation (1) selon l'une quelconque des revendications précédentes, l'opération de ventilation étant ajustée par l'appareil de commande (6) de telle sorte que, pendant l'amenée du fluide (4) et pendant l'évacuation du fluide (4), un débit volumique $F(t)$ (26) varie d'au plus 50 % par rapport à un débit volumique moyen FD (42) dans l'opération de ventilation, au moins pendant 80 % d'une durée de l'opération de ventilation.

**12.** Dispositif de ventilation (1) selon l'une quelconque des revendications 9 à 11, l'appareil de commande (6) étant configuré pour déterminer et ajuster un débit volumique $F(t)$ (26) du fluide (4) au moins pendant une opération de ventilation de l'au moins une voie respiratoire (5) ; l'opération de ventilation ayant lieu dans un intervalle de pression (9) et dans un intervalle de volume (10) ; l'appareil de commande (6) étant configuré pour déterminer, en supposant une résistance de voie respiratoire $R$ (27) de la voie respiratoire (5) du patient, une puissance de perte $PW(t)$ (28) de la voie respiratoire (5) selon $PW(t) = R_1{}^*(F(t))^3 + R_2{}^*(F(t))^2$, avec $R_1 = R$ en Pascal/$(m^3/s)^2$ et $R_2 = R$ en Pascal/$(m^3/s)$ ; au moins un des paramètres suivants étant représenté de manière visuellement reconnaissable par l'appareil de visualisation (17) :

a) la puissance de perte $PW(t)$ (28) ; ou

b) une énergie de perte $E$ (29), à savoir l'intégrale de $PW(t)dt$, c'est-à-dire $\int PW(t)dt$ dans un intervalle de temps ; ou

c) une mesure d'un rapport entre la puissance de perte $PW(t)$ (28) et une puissance de perte critique (30) définie pour un patient déterminé ; ou

d) une mesure d'un rapport entre l'énergie de perte $E$ (29) et une énergie de perte critique (31) définie pour un patient déterminé.

**13.** Dispositif de ventilation (1) selon l'une quelconque des revendications précédentes, l'opération de ventilation étant ajustée par l'appareil de commande (6) de telle sorte qu'un carré d'une vitesse $(s(t))^2$ (32) de l'évolution de la pression P (7) [cmH$_2$O] et du volume V (8) [ml] pendant l'amenée du fluide (4) et pendant l'évacuation du fluide (4), c'est-à-dire $(s(t))^2$ = $(dP/dt)^2$ + $(dV/dt)^2$, par rapport à un carré moyen d'une vitesse sD$^2$ (43) pendant l'opération de ventilation, varie d'au plus 300 % au moins pendant 80 % d'une durée de l'opération de ventilation.

**14.** Dispositif de ventilation (1) selon l'une quelconque des revendications 9 à 13, l'appareil de commande (6) étant configuré pour, au moins pendant une opération de ventilation de l'au moins une voie respiratoire (5), déterminer une évolution d'au moins une courbe volume-pression dans un diagramme volume-pression ; la courbe présentant une première section de courbe (18), V = $f_{ZP}$(P) ou P = $f_{ZV}$(V), et une deuxième section de courbe (19), V = $f_{AP}$(P) ou P = $f_{AV}$(V), la première section de courbe (18) représentant l'évolution du volume amené V (8) et de la pression P (7) pendant l'amenée du fluide (4) dans l'au moins une voie respiratoire (5) et la deuxième section de courbe (19) représentant l'évolution du volume évacué V (8) et de la pression P (7) pendant l'évacuation du fluide (4) de l'au moins une voie respiratoire (5) ; l'opération de ventilation ayant lieu dans un intervalle de pression (9) et dans un intervalle de volume (10) ; l'appareil de commande (6) étant configuré pour déterminer un carré d'une vitesse $(s(t))^2$ (32) de l'évolution de la pression P (7) [cmH$_2$O] et du volume V (8) [ml] pendant l'amenée du fluide (4) et pendant l'évacuation du fluide (4), c'est-à-dire $(s(t))^2$ = $(dP/dt)^2$ + $(dV/dt)^2$, au moins l'un des paramètres suivants étant représenté de manière visuellement reconnaissable par l'appareil de visualisation (17) :

a) le carré de la vitesse s(t), c'est-à-dire $(s(t))^2$ ou
b) l'intégrale de $(s(t))^2$dt, c'est-à-dire $\int (s(t))^2$dt dans un intervalle de temps ; ou
c) une mesure d'un rapport entre le carré de la vitesse $(s(t))^2$ (32) et une vitesse critique au carré définie pour un patient déterminé (33) ; ou
d) une mesure d'un rapport entre l'intégrale de $(s(t))^2$dt et une valeur critique de cette grandeur (34) définie pour un patient déterminé.

# Fig. 1

# Fig. 2

# Fig. 3

# Fig. 4

**Fig. 5**

**Fig. 6**

# Fig. 7

# Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008113752 A1
- WO 2015004229 A1
- US 2007157930 A1
- DE 102016109528